# FASCICULE DE BREVET EUROPEEN

(11) **EP 2 882 452 B1**
(45) Date de publication et mention de la délivrance du brevet: **26.04.2017**
(21) Numéro de dépôt: 13747842.6
(22) Date de dépôt: 09.08.2013
(51) Int. Cl.: A61K 39/395, A61K 9/00, A61K 47/18, C07K 16/24, C07K 16/28, C07K 16/22

(54) **PROCÉDÉ D'ABAISSEMENT DE LA VISCOSITÉ DE SOLUTIONS DE PROTÉINES À CONCENTRATION ÉLEVÉE**
VERFAHREN ZUR VERMINDERUNG DER VISKOSITÄT HOCHKONZENTRIERTER PROTEINLÖSUNGEN
METHOD FOR LOWERING THE VISCOSITY OF HIGH-CONCENTRATION PROTEIN SOLUTIONS

(30) Priorité: 10.08.2012 US 201261682003 P; 10.08.2012 FR 1257775
(43) Date de publication de la demande: 17.06.2015
(73) Titulaire: Adocia, 69003 Lyon (FR)
(72) Inventeur: DAUTY, Emmanuel, F-69004 Lyon (FR); BALLET, Thomas, 92160 ANTONY (FR); SOULA, Rémi, 69330 Meyzieu (FR)
(74) Mandataire: Tripoz, Inès
(86) Numéro de dépôt international: PCT/EP2013/066685
(87) Numéro de publication internationale: WO 2014/023816

(56) Documents cités:
- WO-A1-2011/139718
- WO-A2-2013/028334
- US-A1- 2002 045 571
- BRIAND CONNOLLY ET AL: "Weak Interactions Govern the Viscosity of Concentrated Antibody Solutions: High-Throughput Analysis Using the Diffusion Interaction Parameter", BIOPHYSICAL JOURNAL, BIOPHYSICAL SOCIETY, US, vol. 103, no. 1, 24 avril 2012 (2012-04-24), pages 69-78, XP028398737, ISSN: 0006-3495, DOI: 10.1016/J.BPJ.2012.04.047 [extrait le 2012-05-07]

## Description

L'invention concerne un procédé consistant en l'utilisation de composés réducteurs de viscosité (identifiés ci-après sous l'appellation RV) pour abaisser la viscosité de solutions comprenant au moins une protéine comportant au moins un fragment d'anticorps.

Les anticorps monoclonaux (mAbs) sont une classe de protéines thérapeutiques en pleine expansion pour traiter des pathologies graves comme certains cancers, maladies infectieuses et maladies auto-immunes. A ce jour, plus de 20 mAbs ont été approuvés par la FDA, et environ 20% des protéines thérapeutiques actuellement en développement sont des anticorps.

Les anticorps peuvent être administrés par voie parentérale telle que l'injection intraveineuse (IV), sous-cutanée (SC) ou intramusculaire (IM). Les voies SC et IM permettent de réduire le coût des traitements et améliorent le confort des patients.

Pour les injections SC et IM, le faible volume pouvant être injecté (0,5-2 mL) impose le développement de formulations concentrées en anticorps puisque les doses requises sont classiquement de 100 mg à 1 g pour atteindre un effet thérapeutique. A des concentrations supérieures à 100 mg/mL, les solutions de mAbs sont souvent instables et visqueuses rendant difficiles les étapes de fabrication (étapes de purification, de concentration et de répartition) comme cela est décrit dans la publication de Shire, Current Opinion in Biotechnology 2009, 20, 708-714.

La viscosité des formulations entraîne des problèmes d'injectabilité et rend les administrations douloureuses pour le patient comme cela est décrit dans la publication de Cilurzo et al., AAPS PharmSciTech, 2011, 12(2), 604-9.

Cette viscosité des formulations concentrées en protéines devient donc un véritable challenge comme cela est décrit dans la publication de Adler, American Pharmaceutical Review, February 2012, 15(1).

Même si les facteurs régissant ces augmentations de viscosité sont assez mal décrits et identifiés, de nombreuses publications montrent que l'augmentation de viscosité des solutions d'anticorps à haute concentration résulte d'au moins deux facteurs : des effets stériques (exclusion de volume) et des interactions protéines-protéines comme cela est décrit dans la publication de Saluja & Kalonia, Int J Pharm, 2008, 358(1-2), 1-15.
Mais, comme le seul facteur susceptible d'être modifié par le formulateur est la viscosité des solutions, car les autres facteurs que sont les doses et les calibres des aiguilles ne peuvent dans la plupart des cas pas être modifiés, le besoin d'identifier des excipients réducteurs de viscosité susceptibles d'abaisser la viscosité de solutions à haute concentration de protéines est devenu crucial et stratégique dans l'industrie pharmaceutique.

L'utilisation d'un sel comme le NaCl a été décrite notamment dans la publication de Shire & al. J Pharm Sci 2004, 93(6), 1390-402 ou le brevet US7,666,413 comme permettant d'abaisser la viscosité des solutions de mAb concentrées. Mais cette solution n'est cependant pas universelle et la viscosité de nombreuses formulations de mAb n'est pas réduite ou pas suffisamment réduite par l'addition d'un sel comme le NaCl comme cela est confirmé dans la publication de Guo & al., Pharm Res, 2012, 29(11), 3102-3109.

Il a également été rapporté dans la publication de Guo & al., Pharm Res, 2012, 29(11), 3102-3109 que certains anions hydrophobes permettaient d'abaisser la viscosité de solutions de mAb concentrées et les meilleurs candidats ont été définis dans les conclusions de l'étude structure activité présentée comme devant être hydrophobes, encombrés et aliphatiques. Cependant, ces sels doivent être utilisés à des concentrations d'au moins 250 mM pour atteindre leur activité optimale.

Des acides aminés (brevet US 7,666,413) et des dérivés d'acides aminés (demande de brevet WO2011/139718) ont également été décrits comme permettant de réduire la viscosité de formulations contenant une protéine active de type mAb. En particulier, le sel chlorhydrate d'arginine, composé couramment utilisé dans les procédés de purification et de formulation des protéines, voir par exemple la publication de Frokjaer S., Nat Rev Drug Discov 2005, 4(4), 298-306, peut être considéré comme une des références pour apprécier les abaissements de viscosité. Pour cela, le sel chlorhydrate d'arginine est couramment utilisé à une concentration de l'ordre de 200mM comme cela est rapporté dans le brevet US 7,666,413 ou la publication de Galush & al., J Pharm Sci 2012, 101(3), 1012-20.

Les demandes de brevet WO2012/138958 et WO2012/141978 décrivent une méthode pour réduire la viscosité d'une formulation contenant un polypeptide thérapeutique et du citrate (WO2012/138958) ou de l'acétate (WO2012/141978), la méthode consistant en l'ajout à ladite formulation d'un ou plusieurs acides aminés. Dans ces demandes sont notamment exemptifiées les utilisations de la phénylalanine et de la tyrosine pour abaisser la viscosité de solutions d'anticorps anti-IL5 ou anti-ELR. Cependant, dans la demande WO2012/141978, il est mis en évidence dans un exemple que la tyrosine (0,004% w/v soit 0,2 mM) augmente la viscosité de la solution d'anticorps anti-IL5 (+ 44% d'augmentation).

La demande de brevet WO2013/063510 décrit une formulation pharmaceutiquement stable comprenant un ou plusieurs acides aminés pour stabiliser une protéine de la formulation, et un ou plusieurs acides aminés pour réduire la viscosité de la formulation. Cependant, il est mis en évidence dans un exemple que la phénylalanine (0,2% soit 12 mM) augmente au contraire la viscosité d'une formulation de trastuzumab à 300 mg/mL (+83% d'augmentation).

De façon surprenante la demanderesse a identifié des composés porteurs d'un groupe phényle et d'un groupe ammonium qui sont capables de réduire de manière remarquable la viscosité de solutions difficilement injectables d'au moins une protéine comportant au moins un fragment d'anticorps.

L'invention concerne ainsi l'utilisation d'un composé de formule I, à une concentration dans la formulation finale comprise entre 10 et 250 mM : 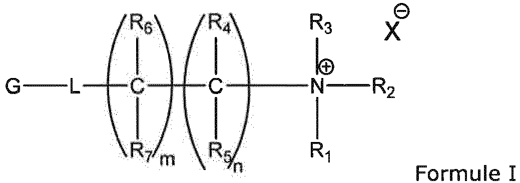 dans laquelle :
▪ G est un groupe phényle ou alkylphényle en C6 à C10, le groupe phényle pouvant être substitué par un ou plusieurs groupements hydroxyles,
▪ L est soit une liaison, soit une fontion choisie dans le groupe constitué de la fonction éther, de la fonction carbamate ou de la fonction amide,
▪ m et n sont des entiers identiques ou différents compris entre 0 et 2 tel que 0 < m + n ≤ 3,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ R₄, R₅, R₆, et R₇, identiques ou différents sont soit un atome d'hydrogène, soit une chaîne comprenant 1 à 6 atomes de carbone, ladite chaîne pouvant comporter un ou plusieurs hétéroatomes N ou O, mais ne porte pas de fonction acide carboxylique,
▪ X⁻ est un anion choisi dans le groupe constitué des halogènures, des carboxylates, des sulfates, phosphates et sulfonates,
▪ les atomes de carbone asymétriques présentent une configuration R ou S. ledit composé ayant une solubilité dans l'eau d'au moins 20 mM à un pH compris entre 5 et 8,

pour abaisser la viscosité d'une solution difficilement injectable d'au moins une protéine comportant au moins un fragment d'anticorps dont la concentration est comprise en 50 et 350 mg/mL et dont le pH est compris entre 5 et 8, d'une valeur d'au moins 15 % par rapport à la viscosité d'une solution d'au moins une protéine comportant au moins un fragment d'anticorps de même concentration et de même pH ne contenant pas ledit composé.

On entend par « solution difficilement injectable d'au moins une protéine comportant au moins un fragment d'anticorps » une solution d'au moins une protéine comportant au moins un fragment d'anticorps dont la viscosité est au moins de 15 cP. Il est généralement admis qu'une telle solution dont la viscosité est au moins de 15 cP est difficilement injectable par voie sous-cutanée à l'aide d'une aiguille d'un calibre d'au moins 29 G. La demanderesse a retenu comme limite le calibre d'au moins 29 G car ce dernier est le calibre supérieur admissible pour que le confort d'injection ne soit pas trop dégradé.

On entend par « protéine comportant au moins un fragment d'anticorps » une protéine choisie parmi les anticorps monoclonaux (mAbs), les anticorps polyclonaux, les protéines de fusion, les nanobodies, les anticorps bispécifiques et les anticorps couplés à des principes actifs cytotoxiques (ADC).

On entend par « anticorps monoclonal » un « anticorps complet », un « fragment d'anticorps » ou un « dérivé d'anticorps » qui possède une spécificité identique et unique i.e. qui ne reconnait qu'un seul type d'épitope sur un antigène donné.

Selon la présente invention, un anticorps peut aussi être appelé immunoglobuline.

On entend par « anticorps complet » un anticorps composé de deux chaînes lourdes identiques (" CH ") et de deux chaînes légères identiques (" CL ") qui sont liées par un pont disulfure. Chaque chaîne est constituée, en position N-terminale, d'une région (ou domaine) variable (codée par les gènes réarrangés V-J pour les chaînes légères et V-D-J pour les chaînes lourdes) spécifique de l'antigène contre lequel l'anticorps est dirigé, et en position C-terminal, d'une région constante, constituée d'un seul domaine CL pour les chaînes légères ou de plusieurs domaines pour les chaînes lourdes. Chaque région variable comprend trois segments appelés " régions déterminant la complémentarité " (" CDRs ") ou " régions hypervariables ", qui sont principalement responsables de la liaison à l'épitope d'un antigène. Les deux chaînes lourdes (H, Heavy) et les deux chaînes légères (L, Light) sont identiques entre elles. La chaîne légère est composée de 2 domaines, un domaine variable V et un domaine constant C, repliés indépendamment l'un de l'autre dans l'espace. On les appelle VL et CL. La chaîne lourde comporte également un domaine V noté VH et 3 ou 4 domaines C noté de CH₁ à CH⁴. Chaque domaine comprend environ 110 acides aminés et est structuré de manière comparable. Les 2 chaînes lourdes sont liées par des ponts disulfures et chaque chaîne lourde est liée à une chaîne légère par un pont disulfure également. La région qui détermine la spécificité de l'anticorps pour l'antigène est portée par les parties variables, alors que les parties constantes peuvent interagir avec les récepteurs Fc des cellules effectrices ou des molécules comme le complément pour médier différentes propriétés fonctionnelles. Le terme "VH" fait référence aux régions variables d'une chaîne lourde d'immunoglobuline d'un anticorps, incluant les chaînes lourdes d'un fragment Fv, scFv, dsFv, Fab, Fab' ou F(ab)'. Le terme " VL " fait référence aux régions variables d'une chaîne légère d'immunoglobuline d'un anticorps, incluant les chaînes légères d'un fragment Fv, scFv, dsFv, Fab, Fab' ou F(ab)'. Par "régions CDR ou CDRs", on entend désigner les régions hypervariables des chaînes lourdes et légères des immunoglobulines comme définies par Kabat et al. (Kabat et al., Sequences of proteins of immunological interest, 5th Ed., U.S. Department of Health and Human Services, NIH, 1991, and later editions, XV). Il existe 3 CDRs de chaîne lourde et 3 CDRs de chaîne légère. Le terme CDR ou CDRs est utilisé ici pour désigner suivant les cas, l'une de ces régions ou plusieurs, voire l'ensemble, de ces régions qui contiennent la majorité des résidus d'acides aminés responsables de la liaison affine de l'anticorps pour l'antigène ou l'épitope qu'il reconnaît. Les régions les plus conservées des domaines variables sont appelés régions ou séquences FR pour "framework" ou régions " charpentes" au nombre de 4 (FR 1 à FR4).

Les anticorps sont subdivisés en 5 classes ou isotypes : IgG, IgA, IgM, IgE et IgD selon la structure des domaines constants des chaînes lourdes, i.e. respectivement chaînes γ, α, µ, ε et δ.

Les classes des IgG et des IgA sont par ailleurs subdivisées en sous-classes selon notamment la taille des régions charnières ainsi que le nombre et la position de ponts disulfures entre chaînes lourdes.

La classe des IgG est subdivisée en 4 sous-classes i.e. IgG1, IgG2, IgG3 et IgG4.

La classe des IgA est quant à elle subdivisée en 2 sous-classes i.e. IgA1 et IgA2.

Dans un mode de réalisation de l'utilisation ou du procédé, la protéine comportant au moins un fragment d'anticorps est un anticorps monoclonal.

Dans un mode de réalisation de l'utilisation ou du procédé, l'anticorps monoclonal est un IgG.

Dans un mode de réalisation de l'utilisation ou du procédé, l'anticorps monoclonal est un IgA.

Dans un mode de réalisation de l'utilisation ou du procédé, l'anticorps monoclonal est un IgM.

Dans un mode de réalisation de l'utilisation ou du procédé, l'anticorps monoclonal est un IgE.

Dans un mode de réalisation de l'utilisation ou du procédé, l'anticorps monoclonal est un IgD.

Dans un mode de réalisation de l'utilisation ou du procédé, l'anticorps monoclonal est un IgG1.

Dans un mode de réalisation de l'utilisation ou du procédé, l'anticorps monoclonal est un IgG2.

Dans un mode de réalisation de l'utilisation ou du procédé, l'anticorps monoclonal est un IgG3.

Dans un mode de réalisation de l'utilisation ou du procédé, l'anticorps monoclonal est un IgG4.

Dans un mode de réalisation de l'utilisation ou du procédé, l'anticorps monoclonal est un IgA1.

Dans un mode de réalisation de l'utilisation ou du procédé, l'anticorps monoclonal est un IgA2.

On entend par «fragment d'anticorps» tout fragment fonctionnel d'anticorps e.g. Fab (Fragment antigen binding), Fv, scFv (single chain Fv), Fc (Fragment cristallisable), F(ab') 2, Fab', scFv-Fc, des polypeptides synthétiques contenant les séquences d'un ou de CDRs, qui possèdent généralement la même spécificité de fixation que l'anticorps dont ils sont issus.

Selon la présente invention, des fragments d'anticorps de l'invention peuvent être obtenus à partir des anticorps par des méthodes telles que la digestion par des enzymes, comme la pepsine ou la papaïne et/ou par clivage des ponts disulfures par réduction chimique. La digestion enzymatique des anticorps par la papaïne génère 2 fragments identiques, qu'on appelle « fragment Fab (Fragment Antigen Binding), et un fragment Fc (fragment cristallisable). Le fragment Fc est le support des fonctions effectrices des immunoglobulines, Par digestion à la pepsine, un fragment F(ab') 2 est généré, où les deux fragments Fab restent liés par deux ponts disulfure, et le fragment Fc est scindé en plusieurs peptides. Le fragment F(ab') 2 est formé de deux fragments Fab', liés par des ponts disulfure intercaténaires pour former un F(ab')2.

Ainsi, l'« anticorps monoclonal » selon l'invention pouvant avantageusement contenir un ou plusieurs de ces fragments, toutes les combinaisons entre les fragments précédemment cités font partie de l'invention.

On entend par « dérivé d'anticorps» tout anticorps, cet anticorps pouvant comprendre une ou plusieurs mutations, substitutions, délétions et/ou additions d'un ou plusieurs résidus d'acides aminés. Un tel ajout, substitution ou délétion peut être localisé à n'importe quelle position dans la molécule. Dans le cas où plusieurs acides aminés ont été ajoutés, substitués ou délétés, toute combinaison d'ajout, de substitution ou de délétion peut être considérée, à condition que l'anticorps résultant présente toujours au moins les propriétés avantageuses de l'anticorps de l'invention.

Selon l'invention, l'« anticorps monoclonal » peut avantageusement être un « anticorps chimérique » ou un « anticorps humanisé ». Par « anticorps chimérique » on entend un anticorps dont les régions variables des chaînes légères et lourdes, ou au moins un domaine ou fragment de ces régions, appartiennent à une espèce différente des régions constantes des chaînes légères et des chaînes lourdes. Par « anticorps humanisé » on entend un anticorps qui contient principalement des séquences immunoglobuline humaines. Ce terme fait généralement référence à une immunoglobuline non humaine qui a été modifiée par incorporation de séquences humaines ou de résidus trouvés dans des séquences humaines.

Les anticorps selon l'invention peuvent être constitués en utilisant les techniques standard de l'ADN recombinant, bien connues de l'homme du métier, par exemple en utilisant les techniques de construction des anticorps « chimériques » décrites par exemple dans Morrison et al., Proc. Natl. Acad. Sci. U.S.A., 1984, 81(21) 6851-55, où la technologie de l'ADN recombinant est utilisée pour remplacer la région constante d'une chaîne lourde et/ou la région constante d'une chaîne légère d'un anticorps provenant d'un mammifère non-humain avec les régions correspondantes d'une Immunoglobuline humaine. De tels anticorps et leur mode de préparation ont également été décrits dans la demande de brevet EP 173 494, dans le document Neuberger, M.S. et al., Nature 312 (5995) : 604-8 (1985), ainsi que dans le document EP 125 023 par exemple. Des méthodes pour générer des anticorps chimériques sont largement disponibles pour l'homme du métier. Par exemple, les chaînes lourdes et légères de l'anticorps peuvent être exprimées séparément en utilisant un vecteur pour chaque chaîne, ou bien être intégrées dans un seul vecteur.

A titre d'exemple on citera parmi les anticorps monoclonaux commercialisés les anticorps monoclonaux suivants :le muromonab-CD3 (commercialisé sous le nom de Orthoclone Okt3^{®}), l'abciximab (commercialisé sous le nom de Reopro^{®}), le rituximab (commercialisé sous les noms de MabThera^{®} et Rituxan^{®}), le basiliximab (commercialisé sous le nom de Simulect^{®}), le daclizumab (commercialisé sous le nom de Zenapax^{®}), le palivizumab (commercialisé sous le nom de Synagis^{®}), l'infliximab (commercialisé sous le nom de Remicade^{®}), le trastuzumab (commercialisé sous le nom de Herceptin^{®}), l'alemtuzumab (commercialisé sous les noms de MabCampath^{®}, Campath-1H^{®}), l'adalimumab (commercialisé sous le nom de Humira^{®}), le tositumomab-I131 (commercialisé sous le nom de Bexxar^{®}), l'efalizumab (commercialisé sous le nom de Raptiva^{®}), le cetuximab (commercialisé sous le nom de Erbitux^{®}), l'omalizumab (commercialisé sous le nom de Xolair®), le bevacizumab (commercialisé sous le nom de Avastin^{®}), le natalizumab (commercialisé sous le nom de Tysabri^{®}), le ranibizumab (commercialisé sous le nom de Lucentis^{®}), le panitumumab (commercialisé sous le nom de Vectibix^{®}), l'eculizumab (commercialisé sous le nom de Soliris^{®}), le golimumab (commercialisé sous le nom de Simponi^{®}), le canakinumab (commercialisé sous le nom de Ilaris^{®}), le catumaxomab (commercialisé sous le nom de Removab^{®}), l'ustekinumab (commercialisé sous le nom de Stelara^{®}), le tocilizumab (commercialisé sous les noms de RoActemra^{®}et Actemra^{®}), l'ofatumumab (commercialisé sous le nom de Arzerra^{®}), le denosumab (commercialisé sous le nom de Prolia^{®}), le belimumab (commercialisé sous le nom de Benlysta^{®}), le raxibacumab (pas encore commercialisé), l'ipilimumab (commercialisé sous le nom de Yervoy^{®}), le certolizumab pegol (commercialisé sous le nom de Cimzia^{®}), l'ibritumomab tiuxetan (commercialisé sous le nom de Zevalin^{®}) et le pertuzumab (commercialisé sous le nom de Perjeta^{®}).

On entend par « anticorps polyclonal » un mélange d'« anticorps complets », un mélange de « fragments d'anticorps » ou un mélange de « dérivés d'anticorps » reconnaissant différents types d'épitopes sur un antigène donné.

Dans un mode de réalisation de l'utilisation ou du procédé, la protéine comportant au moins un fragment d'anticorps est un anticorps polyclonal.

On entend par « protéines de fusion » une construction qui renferme plusieurs protéines ou polypeptides d'origine différente. Cette protéine de fusion est codée par un acide nucléique obtenu par les technologies d'ADN recombinant bien connues de l'homme du métier. Selon la présente invention, la protéine de fusion est constituée par un fragment d'« anticorps monoclonal» tel que précédemment décrit et un fragment d'une « protéique d'intérêt ».

Dans un mode de réalisation de l'utilisation ou du procédé, la protéine comportant au moins un fragment d'anticorps est une protéine de fusion.

A titre d'exemple on citera la protéine de fusion constituée par un fragment d'anticorps monoclonal qui est la région Fc d'une immunoglobuline IgG1 et un fragment d'une protéine d'intérêt qui est le domaine extra-cellulaire du récepteur de protéine CTLA-4 (Cytotoxic T-Lymphocyte Antigen 4), cette protéine de fusion i.e. abatacept, étant commercialisée sous le nom d'Orencia^{®}.

A titre d'exemple on citera également la protéine de fusion constituée par un fragment d'anticorps monoclonal qui est la région Fc d'une IgG1 et un fragment d'une protéine d'intérêt qui est la fraction P75 du récepteur soluble du TNF-alpha, cette protéine de fusion i.e. etanercept étant commercialisée sous le nom Enbrel^{®}.

A titre d'exemple on citera également la protéine de fusion constituée par un fragment d'anticorps monoclonal qui est la région Fc d'une IgG1 et un fragment d'une protéine d'intérêt qui sont les portions extracellulaires de l'IL-1R1 (interleukin-1 receptor component) et de IL-1RAcP (IL-1 receptor accessory protein), cette protéine de fusion i.e. arcalyst étant commercialisée sous le nom de Rilonacept^{®}.

A titre d'exemple on citera également la protéine de fusion constituée par un fragment d'anticorps monoclonal qui sont les régions IgG1 hinge, C(H)2 et C(H)3 domains, et un fragment d'une protéine d'intérêt qui est le domaine extracellulaire de LFA-3, cette protéine de fusion i.e. amevive étant commercialisée sous le nom d'Alefacept®.

On entend par «nanobody» tout domaine variable unique de chaînes lourdes d'immunoglobuline. Les nanobodies sont plus largement décrits dans la publication C. Vincke and S. Muyldermans, in D. Saerens and S. Muyldermans (eds.) Single Domain Antibodies : Methods and Protocols, Methods in Molecular Biology, vol. 911, Springer Science+Business Media, LLC 2012, 15-26; et, Wesolowski & al., Med Microbiol Immunol (2009), 198(3), 157-174.

Dans un mode de réalisation de l'utilisation ou du procédé, la protéine comportant au moins un fragment d'anticorps est un nanobody.

On entend par « anticorps bispécifique» (aussi appelé « anticorps bifonctionnel» ou « diabody ») tout fragment d'immunoglobuline comprenant 2 sites de présentation à l'antigène. Les anticorps bifonctionnels sont plus largement décrits dans la publication Hollinger et al., Proc. Natl. Acad. Sci. USA 90(4): 6444-6448 (1993).

Dans un mode de réalisation de l'utilisation ou du procédé, la protéine comportant au moins un fragment d'anticorps est un anticorps bispécifique.

On entend par « anticorps couplé à un principe actif cytotoxique » un « anticorps monoclonal» tel que précédemment décrit couplé à un principe actif cytotoxique.

A titre d'exemples de principes actifs cytotoxiques, on citera notamment ozogamicin et vedotin.

Dans un mode de réalisation de l'utilisation ou du procédé, la protéine comportant au moins un fragment d'anticorps est un anticorps couplé à un principe actif cytotoxique.

A titre d'exemple l'anticorps couplé à un principe actif cytotoxique est l'anticorps brentuximab couplé au principe actif cytotoxique vedotin. Cet anticorps couplé à ce principe actif cytotoxique est commercialisé sous le nom d'Adcetris^{®}.

Dans un mode de réalisation, l'invention concerne également l'utilisation d'un composé de formule I, à une concentration dans la formulation finale comprise entre 10 et 250 mM pour abaisser la viscosité d'une solution difficilement injectable d'au moins une protéine comportant au moins un fragment d'anticorps dont la concentration est comprise en 50 et 350 mg/mL et dont le pH est compris entre 5 et 8, d'une valeur comprise d'au moins 15 % par rapport à la viscosité d'une solution d'au moins une protéine comportant au moins un fragment d'anticorps de même concentration et de même pH ne contenant pas ledit composé.

Dans un mode de réalisation, l'invention concerne également l'utilisation d'un composé de formule I, à une concentration dans la formulation finale comprise entre 10 et 250 mM pour abaisser la viscosité d'une solution difficilement injectable d'au moins une protéine comportant au moins un fragment d'anticorps dont la concentration est comprise en 50 et 350 mg/mL et dont le pH est compris entre 5 et 8, d'une valeur comprise entre 15 et 95% par rapport à la viscosité d'une solution d'au moins une protéine comportant au moins un fragment d'anticorps de même concentration et de même pH ne contenant pas ledit composé.

Dans un mode de réalisation, l'invention concerne également l'utilisation d'un composé de formule I, à une concentration dans la formulation finale comprise entre 100 et 200 mM pour abaisser la viscosité d'une solution difficilement injectable d'au moins une protéine comportant au moins un fragment d'anticorps dont la concentration est comprise en 50 et 350 mg/mL et dont le pH est compris entre 5 et 8, d'une valeur d'au moins 15% par rapport à la viscosité d'une solution d'au moins une protéine comportant au moins un fragment d'anticorps de même concentration et de même pH ne contenant pas ledit composé.

Dans un mode de réalisation, l'invention concerne également l'utilisation d'un composé de formule I, à une concentration dans la formulation finale comprise entre 100 et 200 mM pour abaisser la viscosité d'une solution difficilement injectable d'au moins une protéine comportant au moins un fragment d'anticorps dont la concentration est comprise en 50 et 350 mg/mL et dont le pH est compris entre 5 et 8, d'une valeur comprise entre 15 et 95% par rapport à la viscosité d'une solution d'au moins une protéine comportant au moins un fragment d'anticorps de même concentration et de même pH ne contenant pas ledit composé.

Elle concerne également le procédé d'abaissement de la viscosité consistant à préparer une solution comprenant un composé de formule I et une protéine comportant au moins un fragment d'anticorps dont la concentration est comprise entre 50 et 350 mg/mL et dont le pH est compris entre 5 et 8.

Dans un mode de réalisation, le procédé selon l'invention est caractérisé en ce que l'on prépare une solution comprenant un composé de formule I et une protéine comportant au moins un fragment d'anticorps dont la concentration est comprise en 50 et 350 mg/mL et dont le pH est compris entre 5 et 8 pour abaisser la viscosité de cette solution d'une valeur d'au moins 15% par rapport à la viscosité d'une solution d'au moins une protéine comportant au moins un fragment d'anticorps de même concentration et de même pH ne contenant pas ledit composé.

Dans un mode de réalisation, le procédé selon l'invention est caractérisé en ce que l'on prépare une solution comprenant un composé de formule I et une protéine comportant au moins un fragment d'anticorps dont la concentration est comprise en 50 et 350 mg/mL et dont le pH est compris entre 5 et 8 pour abaisser la viscosité de cette solution d'une valeur comprise entre 15 et 95% par rapport à la viscosité d'une solution d'au moins une protéine comportant au moins un fragment d'anticorps de même concentration et de même pH ne contenant pas ledit composé.

Dans un mode de réalisation, le procédé selon l'invention est caractérisé en ce que la solution finale à une concentration en composé de formule I comprise entre 10 et 250 mM.

Dans un mode de réalisation, le procédé selon l'invention est caractérisé en ce que la solution finale à une concentration en composé de formule I comprise entre 100 et 200 mM.

Dans un mode de réalisation, G est un groupe phényle.

Dans un mode de réalisation, G est un groupe phényle substitué par un ou plusieurs groupements hydroxyles.

Dans un mode de réalisation, R₄ et R₆, identiques ou différents sont soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₅ et R₇, identiques ou différents sont soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, au moins un groupe R₄, R₅, R₆ ou R₇ n'est pas un atome d'hydrogène et comprend une fonction amine-N-oxyde.

Dans un mode de réalisation, au moins un groupe R₄, R₅, R₆ ou R₇ n'est pas un atome d'hydrogène et comprend une fonction amine-N-oxyde et une fonction amide.

Dans un mode de réalisation, les fonctions amine-N-oxyde sont salifiées, par exemple sous la forme de sel de chlorhydrate :

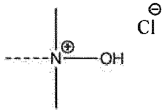

Il a été choisi de représenter les fonction ammonium sous la forme « NR₄⁺.X⁻», il est cependant évident que, lorsqu'au moins un des groupes R est un atome d'hydrogène, 100% des espèces en présence ne sont pas obligatoirement sous cette forme dans certaines conditions de pH. Dans la gamme de pH 5 à 8 selectionné dans l'invention, il est possible qu'une partie des espèces en présence ne soit pas sous forme de sel à un pH donné.

Dans un mode de réalisation de l'utilisation ou du procédé, le pH est compris entre 5 et 6,5.

Dans un mode de réalisation de l'utilisation ou du procédé, le pH est compris entre 5,5 et 6,5.

Dans un mode de réalisation de l'utilisation ou du procédé, le pH est compris entre 6 et 8.

Dans un mode de réalisation de l'utilisation ou du procédé, le pH est compris entre 6 et 7,5.

Dans un mode de réalisation de l'utilisation ou du procédé, le pH est compris entre 6 et 7.

Une concentration exprimée en M est une concentration en mol/L.

Une concentration exprimée en mM est une concentration en mmol/L.

Dans un mode de réalisation de l'utilisation ou du procédé, la concentration du composé de formule I dans la formulation finale est comprise entre 10 et 200 mM.

Dans un mode de réalisation de l'utilisation ou du procédé, la concentration du composé de formule I dans la formulation finale est comprise entre 25 et 200 mM.

Dans un mode de réalisation de l'utilisation ou du procédé, la concentration du composé de formule I dans la formulation finale est comprise entre 50 et 200 mM.

Dans un mode de réalisation de l'utilisation ou du procédé, la concentration du composé de formule I dans la formulation finale est comprise entre 50 et 175 mM.

Dans un mode de réalisation de l'utilisation ou du procédé, la concentration du composé de formule I dans la formulation finale est comprise entre 100 et 250 mM.

Dans un mode de réalisation de l'utilisation ou du procédé, la concentration du composé de formule I dans la formulation finale est comprise entre 100 et 200 mM.

Dans un mode de réalisation de l'utilisation ou du procédé, la concentration du composé de formule I dans la formulation finale est comprise entre 100 et 175 mM.

Dans un mode de réalisation de l'utilisation ou du procédé, la concentration en protéine comportant au moins un fragment d'anticorps dans la formulation finale est comprise entre 100 et 350 mg/mL.

Dans un mode de réalisation de l'utilisation ou du procédé, la concentration en protéine comportant au moins un fragment d'anticorps dans la formulation finale est comprise entre 100 et 300 mg/mL.

Dans un mode de réalisation de l'utilisation ou du procédé, la concentration en protéine comportant au moins un fragment d'anticorps dans la formulation finale est comprise entre 100 et 250 mg/mL.

Dans un mode de réalisation de l'utilisation ou du procédé, la concentration en protéine comportant au moins un fragment d'anticorps dans la formulation finale est comprise entre 150 et 300 mg/mL.

Dans un mode de réalisation de l'utilisation ou du procédé, la concentration en protéine comportant au moins un fragment d'anticorps dans la formulation finale est comprise entre 150 et 250 mg/mL.

Dans un mode de réalisation de l'utilisation ou du procédé, la viscosité est abaissée d'une valeur d'au moins 15%.

Dans un mode de réalisation de l'utilisation ou du procédé, la viscosité est abaissée d'une valeur comprise entre 15 et 95%.

Dans un mode de réalisation de l'utilisation ou du procédé, la viscosité est abaissée d'une valeur comprise entre 15 et 90%.

Dans un mode de réalisation de l'utilisation ou du procédé, la viscosité est abaissée d'une valeur comprise entre 20 et 80%.

Dans un mode de réalisation de l'utilisation ou du procédé, la viscosité est abaissée d'une valeur comprise entre 30 et 70%.

Dans un mode de réalisation de l'utilisation ou du procédé, la viscosité est abaissée d'une valeur comprise entre 35 et 65%.

Dans un mode de réalisation de l'utilisation ou du procédé, la viscosité est abaissée d'une valeur comprise entre 30 et 65%.

Dans un mode de réalisation de l'utilisation ou du procédé, la viscosité est abaissée d'une valeur comprise entre 25 et 65%.

Dans un mode de réalisation de l'utilisation ou du procédé, la viscosité est abaissée d'une valeur comprise entre 20 et 65%.

Dans un mode de réalisation de l'utilisation ou du procédé, le composé de formule I est choisi parmi les composés dont l'anion X⁻ est un carboxylate choisi dans le groupe constitué par les acétates, les succinates ou les citrates.

Dans un mode de réalisation de l'utilisation ou du procédé, le composé de formule I est choisi parmi les composés dont l'anion X⁻ est choisi dans le groupe des halogénures.

Dans un mode de réalisation de l'utilisation ou du procédé, le composé de formule I est choisi parmi les composés dont l'anion X⁻ est un chlorhydrate.

Dans un mode de réalisation de l'utilisation ou du procédé, le composé est choisi parmi les composés de formule I dans laquelle les groupes R₁, R₂ et R₃, identiques ou différents, ne sont pas un atome d'hydrogène.

Dans un mode de réalisation, les groupes R₁, R₂ et R₃, identiques ou différents, sont choisis dans le groupe constitué de l'atome d'hydrogène, le méthyl, l'éthyl, le propyl, le *n*-butyl, le *sec*-butyl, l'isobutyl le 2-hydroxyéthyl, le 3-hydroxypropyl, le 4-hydroxybutyl et le 1-méthoxypropyl.

Dans un mode de réalisation, R₁ = R₂ = R₃ = H.

Dans un mode de réalisation, R₁ = R₂ = R₃ = CH₃.

Dans un mode de réalisation, R₁ = R₂ = H et R₃ = CH₂CH₂OH.

Dans un mode de réalisation de l'utilisation ou du procédé, le composé est choisi parmi les composés de formule II : 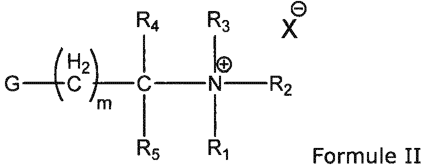 dans laquelle :
▪ G est un groupement phényle ou alkylphényle en C6 à C10, le groupe phényle pouvant être substitué par un ou plusieurs groupements hydroxyles,
▪ m est un entier égal à 0 ou 1,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₄ est une chaîne comprenant de 1 à 6 atomes de carbone, ladite chaine pouvant comporter un ou plusieurs hétéroatomes N ou O, mais ne porte pas de fonction acide carboxylique,
▪ R₅ est soit un atome d'hydrogène, soit une chaine comprenant 1 à 6 atomes de carbone ladite chaîne pouvant comporter un ou plusieurs hétéroatomes N ou O, mais ne porte pas de fonction acide carboxylique,
▪ X⁻ est un anion choisi dans le groupe constitué des halogénures, des carboxylates, des sulfates, phosphates et sulfonates,
▪ les atomes de carbone asymétriques présentent une configuration R ou S.

Dans un mode de réalisation, G est un groupe phényle.

Dans un mode de réalisation, G est un groupe phényle substitué par un ou plusieurs groupements hydroxyles.

Dans un mode de réalisation R₄ est soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation R₄ est une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₄ n'est pas un atome d'hydrogène et comprend une fonction amine-N-oxyde.

Dans un mode de réalisation, R₄ n'est pas un atome d'hydrogène et comprend une fonction amine-N-oxyde et une fonction amide.

Dans un mode de réalisation, R₅ = H et R₄ =

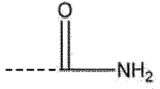

Dans un mode de réalisation, R₅ = H et R₄ = -CH₂OH

Dans un mode de réalisation, R₅ = H et R₄ =

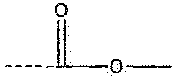

Dans un mode de réalisation, R₅ = H et R₄ =

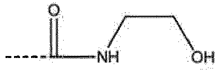

Dans un mode de réalisation, R₅ = H et R₄ =

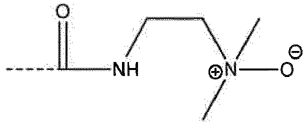

Dans un mode de réalisation de l'utilisation ou du procédé, le composé est choisi parmi les composés de formule IX : 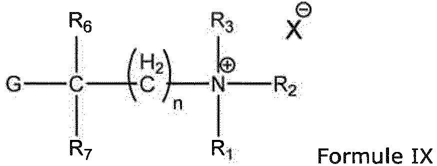 dans laquelle :
▪ G est un groupement phényle ou alkylphényle en C6 à C10, le groupe phényle pouvant être substitué par un ou plusieurs groupements hydroxyles,
▪ n est un entier égal à 1,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₆ est une chaîne comprenant 1 à 6 atomes de carbone, ladite chaine pouvant comporter un ou plusieurs hétéroatomes N ou O, mais ne porte pas de fonction acide carboxylique,
▪ R₇ est soit un atome d'hydrogène, soit une chaine comprenant 1 à 6 atomes de carbone ladite chaîne pouvant comporter un ou plusieurs hétéroatomes N ou O, mais ne porte pas de fonction acide carboxylique,
▪ X⁻ est un anion choisi dans le groupe constitué des halogénures, des carboxylates, sulfates, phosphates et sulfonates,
▪ les atomes de carbone asymétriques présentent une configuration R ou S.

Dans un mode de réalisation, G est un groupe phényle.

Dans un mode de réalisation, G est un groupe phényle substitué par un ou plusieurs groupements hydroxyles.

Dans un mode de réalisation R₆ est soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation R₆ est une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₆ n'est pas un atome d'hydrogène et comprend une fonction amine-N-oxyde.

Dans un mode de réalisation, R₆ n'est pas un atome d'hydrogène et comprend une fonction amine-N-oxyde et une fonction amide.

Dans un mode de réalisation, R₇ = H et R₆ =

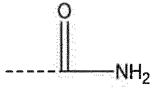

Dans un mode de réalisation, R₇ = H et R₆ = -CH₂OH

Dans un mode de réalisation, R₇ = H et R₆ =

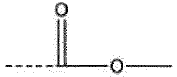

Dans un mode de réalisation, R₇ = H et R₆ =

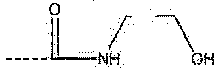

Dans un mode de réalisation, R₇ = H et R₆ =

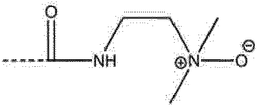

Dans un mode de réalisation de l'utilisation ou du procédé, le composé est choisi parmi les composés de formule III : 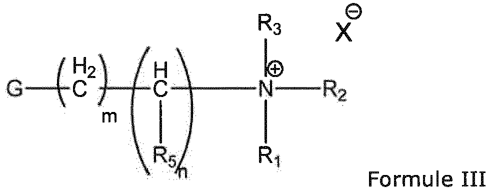 dans laquelle :
▪ G est un groupe phényle ou alkylphényle en C6 à C10, le groupe phényle pouvant être substitué par un ou plusieurs groupements hydroxyles,
▪ m et n sont des entiers identiques ou différents compris entre 0 et 2 tel que 0 < m + n ≤ 3,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₅ est soit un atome d'hydrogène, soit une chaîne alkyle de 1 à 2 atomes de carbone,
▪ X⁻ est un anion choisi dans le groupe constitué des halogènures, des carboxylates, des sulfates, phosphates et sulfonates,
▪ Les atomes de carbone asymétriques présentent une configuration R ou S.

Dans un mode de réalisation de l'utilisation ou du procédé, G est un groupe phényle ou alkylphényle en C6 à C10 dans lequel le groupe phényle est non substitué.

Dans un mode de réalisation, G est un groupe phényle.

Dans un mode de réalisation, G est un groupe phényle substitué par un ou plusieurs groupements hydroxyles.

Dans un mode de réalisation de l'utilisation ou du procédé, le composé est choisi parmi les composés de formule IV : 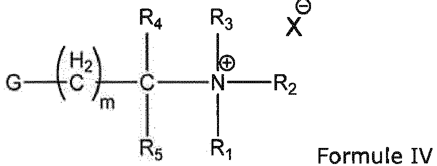 dans laquelle :
▪ G est un groupement phényle ou alkylphényle en C6 à C10, le groupe phényle étant substitué par un ou plusieurs groupements hydroxyles,
▪ m est un entier égal à 0 ou 1,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₄ est une chaîne comprenant 1 à 6 atomes de carbone, ladite chaine pouvant comporter un ou plusieurs hétéroatomes N ou O, mais ne porte pas de fonction acide carboxylique,
▪ R₅ est soit un atome d'hydrogène, soit une chaine comprenant 1 à 6 atomes de carbone ladite chaîne pouvant comporter un ou plusieurs hétéroatomes N ou O, mais ne porte pas de fonction acide carboxylique,
▪ X- est un anion choisi dans le groupe constitué des halogénures, des carboxylates, des sulfates, phosphates et sulfonates,
▪ les atomes de carbone asymétriques présentent une configuration R ou S.

Dans un mode de réalisation R₄ est soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation R₄ est une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₄ n'est pas un atome d'hydrogène et comprend une fonction amine-N-oxyde.

Dans un mode de réalisation, au moins un groupe R₄ n'est pas un atome d'hydrogène et comprend une fonction amine-N-oxyde et une fonction amide.

Dans un mode de réalisation, R₅ = H et R₄ =

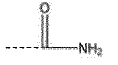

Dans un mode de réalisation, R₅ = H et R₄ = -CH₂OH

Dans un mode de réalisation, R₅ = H et R₄ =

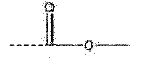

Dans un mode de réalisation, R₅ = H et R₄ =

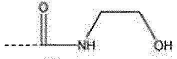

Dans un mode de réalisation, R₅ = H et R₄ =

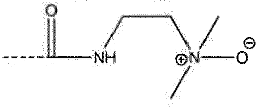

Dans un mode de réalisation de l'utilisation ou du procédé, le composé est choisi parmi les composés de formule V : 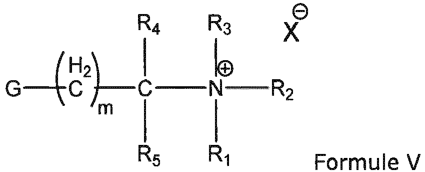 dans laquelle :
▪ G est un groupement phényle ou alkylphényle en C6 à C10,
▪ m est un entier égal à 0 ou 1,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₄ est une chaîne comprenant 1 à 6 atomes de carbone, ladite chaine pouvant comporter un ou plusieurs hétéroatomes N ou O, mais ne porte pas de fonction acide carboxylique,
▪ R₅ est soit un atome d'hydrogène, soit une chaîne comprenant 1 à 6 atomes de carbone, ladite chaine pouvant comporter un ou plusieurs hétéroatomes N ou O, mais ne porte pas de fonction acide carboxylique,
▪ X⁻ est un anion choisi dans le groupe constitué des halogènures, acétates, sulfates, phosphates et sulfonates,
▪ les atomes de carbone asymétriques présentent une configuration R ou S.

Dans un mode de réalisation, G est un groupe phényle.

Dans un mode de réalisation R₄ est soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation R₄ est une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₄ n'est pas un atome d'hydrogène et comprend une fonction amine-N-oxyde.

Dans un mode de réalisation, au moins un groupe R₄ n'est pas un atome d'hydrogène et comprend une fonction amine-N-oxyde et une fonction amide.

Dans un mode de réalisation, R₅ = H et R₄ =

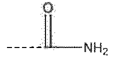

Dans un mode de réalisation, R₅ = H et R₄ = -CH₂OH

Dans un mode de réalisation, R₅ = H et R₄ =

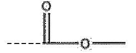

Dans un mode de réalisation, R₅ = H et R₄ =

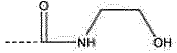

Dans un mode de réalisation, R₅ = H et R₄ =

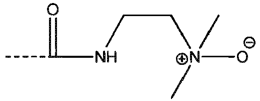

Dans un mode de réalisation de l'utilisation ou du procédé, le composé est choisi parmi les composés de formule XII : 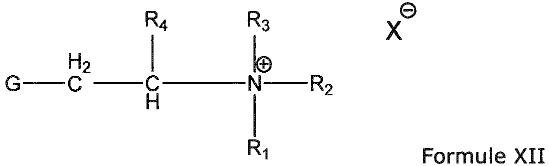 dans laquelle :
▪ G est un groupe phényle ou alkylphényle en C6 à C10, le groupe phényle pouvant être substitué par un ou plusieurs groupements hydroxyles,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ R₄, est une chaîne comprenant 1 à 6 atomes de carbone, ladite chaine pouvant comporter un ou plusieurs hétéroatomes N ou O, mais ne porte pas de fonction acide carboxylique,
▪ X⁻ est au moins un anion choisi dans le groupe constitué des halogènures, des carboxylates, des sulfates, phosphates et sulfonates,
▪ Les atomes de carbone asymétriques présentent une configuration R ou S.

Dans un mode de réalisation, G est un groupe phényle.

Dans un mode de réalisation, G est un groupe phényle substitué par un ou plusieurs groupements hydroxyles.

Dans un mode de réalisation R₄ est une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₄ comprend une fonction amine-N-oxyde.

Dans un mode de réalisation, R₄ comprend une fonction amine-N-oxyde et une fonction amide.

Dans un mode de réalisation, R₄ =

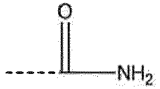

Dans un mode de réalisation, R₄ = -CH2OH

Dans un mode de réalisation, R₄ =

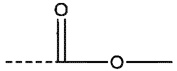

Dans un mode de réalisation, R₄ =

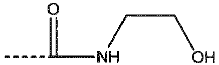

Dans un mode de réalisation, R₄ =

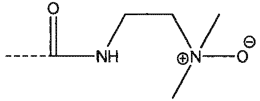

Dans un mode de réalisation de l'utilisation ou du procédé, le composé est choisi parmi les composés de formule XIII : 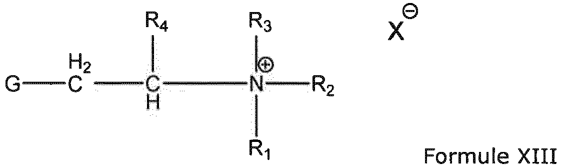 dans laquelle :
▪ G est un groupe phényle ou alkylphényle en C6 à C10, le groupe phényle pouvant être substitué par un ou plusieurs groupements hydroxyles,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ R₄, est une chaîne comprenant 1 à 6 atomes de carbone, ladite chaine comportant un ou plusieurs hétéroatomes N ou O, une fonction amine-N-oxyde, mais ne porte pas de fonction acide carboxylique,
▪ X- est au moins un anion choisi dans le groupe constitué des halogènures, des carboxylates, des sulfates, phosphates et sulfonates,
▪ Les atomes de carbone asymétriques présentent une configuration R ou S.

Dans un mode de réalisation, G est un groupe phényle.

Dans un mode de réalisation, G est un groupe phényle substitué par un ou plusieurs groupements hydroxyles.

Dans un mode de réalisation R₄ est une chaîne comprenant 1 à 6 atomes de carbone, ladite chaîne comportant une fonction amine-N-oxyde et pouvant comporter une ou plusieurs fonctions choisies dans le groupe constitué par les fonctions hydroxyle, amide ou ester.

Dans un mode de réalisation, R₄ =

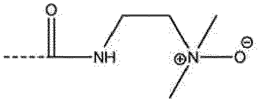

Dans un mode de réalisation de l'utilisation ou du procédé, le composé est choisi parmi les composés de formule XIV : 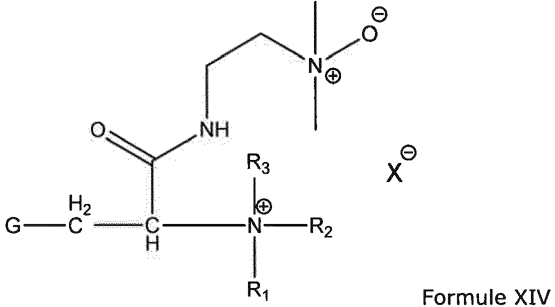 dans laquelle :
▪ G est un groupe phényle ou alkylphényle en C6 à C10, le groupe phényle pouvant être substitué par un ou plusieurs groupements hydroxyles,
▪ R₁, R_{2,} R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ X⁻ est au moins un anion choisi dans le groupe constitué des halogènures, des carboxylates, des sulfates, phosphates et sulfonates,
▪ Les atomes de carbone asymétriques présentent une configuration R ou S.

Dans un mode de réalisation, G est un groupe phényle.

Dans un mode de réalisation, G est un groupe phényle substitué par un ou plusieurs groupements hydroxyles.

Dans un mode de réalisation de l'utilisation ou du procédé, le composé est un composé de formule XV : 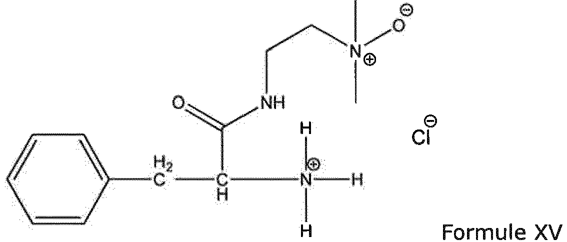 dans laquelle :
▪ Les atomes de carbone asymétriques présentent une configuration R ou S.

Dans un mode de réalisation de l'utilisation ou du procédé, le composé est choisi parmi les composés de formule X : 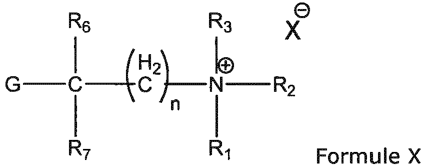 dans laquelle :
▪ G est un groupement phényle ou alkylphényle en C6 à C10, le group phényle étant substitué par un ou plusieurs groupements hydroxyles,
▪ n est un entier égal à 1,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₆, est une chaîne comprenant 1 à 6 atomes de carbone, ladite chaine pouvant comporter un ou plusieurs hétéroatomes N ou O, mais ne porte pas de fonction acide carboxylique,
▪ R₇ est soit un atome d'hydrogène, soit une chaine comprenant 1 à 6 atomes de carbone ladite chaîne pouvant comporter un ou plusieurs hétéroatomes N ou O, mais ne porte pas de fonction acide carboxylique,
▪ X⁻ est un anion choisi dans le groupe constitué des halogénures, des carboxylates, des sulfates, phosphates et sulfonates,
▪ les atomes de carbone asymétriques présentent une configuration R ou S.

Dans un mode de réalisation R₆ est soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation R₆ est une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₆ n'est pas un atome d'hydrogène et comprend une fonction amine-N-oxyde.

Dans un mode de réalisation, R₆ n'est pas un atome d'hydrogène et comprend une fonction amine-N-oxyde et une fonction amide.

Dans un mode de réalisation, R₇ = H et R₆ =

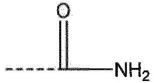

Dans un mode de réalisation, R₇ = H et R₆ = -CH2OH

Dans un mode de réalisation, R₇ = H et R₆ =

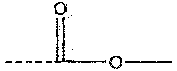

Dans un mode de réalisation, R₇ = H et R₆ =

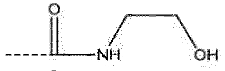

Dans un mode de réalisation, R₇ = H et R₆ =

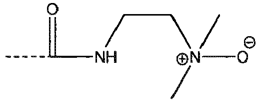

Dans un mode de réalisation de l'utilisation ou du procédé, le composé est choisi parmi les composés de formule XI : 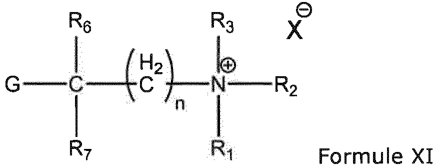 dans laquelle :
▪ G est un groupement phényle ou alkylphényle en C6 à C10,
▪ n est un entier égal à 1,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₆ est une chaîne comprenant 1 à 6 atomes de carbone, ladite chaine pouvant comporter un ou plusieurs hétéroatomes N ou O, mais ne porte pas de fonction acide carboxylique,
▪ R₇ est soit un atome d'hydrogène, soit une chaine comprenant 1 à 6 atomes de carbone ladite chaîne pouvant comporter un ou plusieurs hétéroatomes N ou O, mais ne porte pas de fonction acide carboxylique,
▪ X- est un anion choisi dans le groupe constitué des halogénures, des carboxylates, des sulfates, phosphates et sulfonates,
▪ les atomes de carbone asymétriques sont de configuration R ou S.

Dans un mode de réalisation, G est un groupe phényle.

Dans un mode de réalisation R₆ est soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation R₆ est une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, R₆ n'est pas un atome d'hydrogène et comprend une fonction amine-N-oxyde.

Dans un mode de réalisation, au moins un groupe R₆ n'est pas un atome d'hydrogène et comprend une fonction amine-N-oxyde et une fonction amide.

Dans un mode de réalisation, R₇ = H et R₆ =

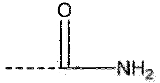

Dans un mode de réalisation, R₇ = H et R₆ = -CH2OH

Dans un mode de réalisation, R₇ = H et R₆ =

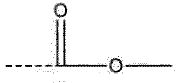

Dans un mode de réalisation, R₇ = H et R₆ =

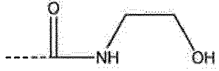

Dans un mode de réalisation, R₇ = H et R₆ =

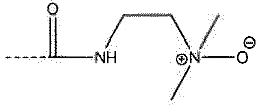

Dans un mode de réalisation de l'utilisation ou du procédé, le composé est choisi parmi les composés de formule VII : 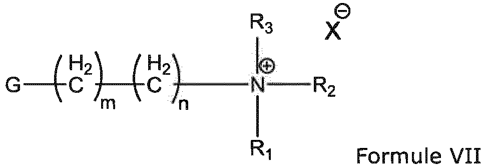 dans laquelle :
▪ G est un groupement phényle ou alkylphényle en C6 à C10, le groupe phényle pouvant être substitué par un ou plusieurs groupements hydroxyles,
▪ m et n sont des entiers identiques ou différents compris entre 0 et 2 tel que 0 < m + n ≤ 3,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ X⁻ est un anion choisi dans le groupe constitué des halogénures, des carboxylates, des sulfates, phosphates et sulfonates,
▪ les atomes de carbone asymétriques présentent une configuration R ou S.

Dans un mode de réalisation, G est un groupe phényle.

Dans un mode de réalisation, G est un groupe phényle substitué par un ou plusieurs groupements hydroxyles.

Dans un mode de réalisation de l'utilisation ou du procédé, m = n = 1.

Dans un mode de réalisation de l'utilisation ou du procédé, m = 0 et n = 1.

Dans un mode de réalisation de l'utilisation ou du procédé, le composé est choisi parmi les composés de formule VIII : 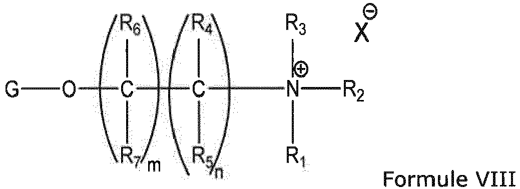 dans laquelle :
▪ G est un groupement phényle ou alkylphényle en C6 à C10, le groupe phényle pouvant être substitué par un ou plusieurs groupements hydroxyles,
▪ m et n sont des entiers identiques ou différents compris entre 0 et 2 tel que 0 < m + n ≤ 3,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O, mais ne porte pas de fonction acide carboxylique,
▪ R₄, R₅, R₆ et R₇, identiques ou différents sont soit un atome d'hydrogène, soit une chaîne comprenant 1 à 6 atomes de carbone, ladite chaine pouvant comporter un ou plusieurs hétéroatomes N ou O, mais ne porte pas de fonction acide carboxylique,
▪ X⁻ est un anion choisi dans le groupe constitué des halogénures, des carboxylates, des sulfates, phosphates et sulfonates,
▪ les atomes de carbone asymétriques présentent une configuration R ou S.

Dans un mode de réalisation, G est un groupe phényle.

Dans un mode de réalisation, G est un groupe phényle substitué par un ou plusieurs groupements hydroxyles.

Dans un mode de réalisation, R₄ et R₆, identiques ou différents sont soit un atome d'hydrogène, soit une chaîne comprenant 1 à 6 atomes de carbone, ladite chaîne pouvant comporter une ou plusieurs fonctions choisies dans le groupe constitué par les fonctions hydroxyle, amide ou ester.

Dans un mode de réalisation, au moins un groupe R₄, R₅, R₆ ou R₇ n'est pas un atome d'hydrogène et comprend une fonction amine-N-oxyde.

Dans un mode de réalisation, au moins un groupe R₄, R₅, R₆ ou R₇ n'est pas un atome d'hydrogène et comprend une fonction amine-N-oxyde et une fonction amide.

Dans un mode de réalisation, R₅ = R₆ = R₇ = H et R₄ =

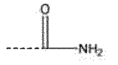

Dans un mode de réalisation, R₅ = R₆ = R₇ = H et R₄ = -CH₂OH

Dans un mode de réalisation, R₅ = R₆ = R₇ = H et R₄ =

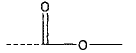

Dans un mode de réalisation, R₅ = R₆ = R₇ = H et R₄ =

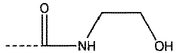

Dans un mode de réalisation, R₅ = R₆ = R₇ = H et R₄

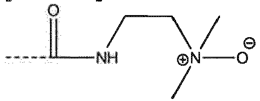

Dans un mode de réalisation, R₅ = R₄ = R₇ = H et R₆ =

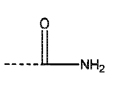

Dans un mode de réalisation, R₅ = R₄ = R₇ = H et R₆ = -CH₂OH

Dans un mode de réalisation, R₅ = R₄ = R₇ = H et R₆ =

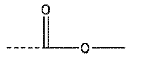

Dans un mode de réalisation, R₅ = R₄ = R₇ = H et R₆ =

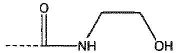

Dans un mode de réalisation, R₅ = R₄ = R₇ = H et R₆

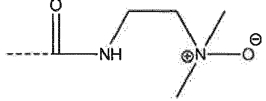

Dans un mode de réalisation de l'utilisation ou du procédé, le composé de formule I est le sel chlorhydrate de la phénylalaninamide et R₁ = R₂ = R₃ = H, L est une liaison, G = C₆H₅, m = n = 1, R₄ = CONH₂, R₅ = R₆ = R₇ = H, et X⁻ = Cl⁻ selon la formule :

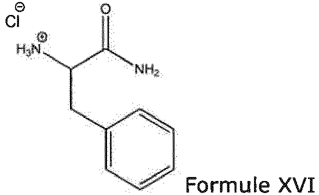

Dans un mode de réalisation de l'utilisation ou du procédé, le composé de formule I est le racémique du sel chlorhydrate de la phénylalaninamide.

Dans un mode de réalisation de l'utilisation ou du procédé, le composé de formule I est le sel chlorhydrate de la L-phénylalaninamide.

Dans un mode de réalisation de l'utilisation ou du procédé, le composé de formule I est le sel chlorhydrate de la D-phénylalaninamide.

Dans un mode de réalisation de l'utilisation ou du procédé, le composé de formule I est le sel chlorhydrate du 2-amino-3-phényl-1-propanol et R₁ = R₂ = R₃ = H, L est une liaison, G = C₆H₅, m = n = 1, R₄ = CH₂OH, R₅ = R₆ = R₇ = H, et X⁻ = Cl⁻ selon la formule :

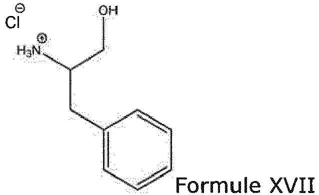

Dans un mode de réalisation de l'utilisation ou du procédé, le composé de formule I est le racémique du sel chlorhydrate du 2-amino-3-phényl-1-propanol.

Dans un mode de réalisation de l'utilisation ou du procédé, le composé de formule I est le sel chlorhydrate du (S)-(-)-2-amino-3-phényl-1-propanol.

Dans un mode de réalisation de l'utilisation ou du procédé, le composé de formule I est le sel chlorhydrate du (R)-(+)-2-amino-3-phényl-1-propanol.

Dans un mode de réalisation de l'utilisation ou du procédé, le composé de formule I est le sel chlorhydrate de l'ester méthylique de la phénylalanine et R₁ = R₂ = R₃ = H, L est une liaison, G = C₆H₅, m = n = 1, R₄ = COOCH₃, R₅ = R₆ = R₅ = H et X⁻ = Cl⁻ selon la formule :

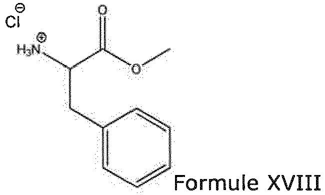

Dans un mode de réalisation de l'utilisation ou du procédé, le composé de formule I est le racémique du sel chlorhydrate de l'ester méthylique de la phénylalanine.

Dans un mode de réalisation de l'utilisation ou du procédé, le composé de formule I est le sel chlorhydrate de l'ester méthylique de la L-phénylalanine.

Dans un mode de réalisation de l'utilisation ou du procédé, le composé de formule I est le sel chlorhydrate de l'ester méthylique de la D-phénylalanine.

Dans un mode de réalisation de l'utilisation ou du procédé, le composé de formule I est le sel dichlorhydrate d'alpha-amino-N-[2-(N-hydroxy-N,N-diméthylaminium)éthyl] de benzene propanamide et R₁ = R₂ = R₃ = H, L est une liaison, G = C₆H₅, m = n = 1, R₄ = CONHCH₂CH₂N(CH₃)₂O, R₅ = R₆ = R₇ = H et X⁻ = Cl⁻ de formule XV:

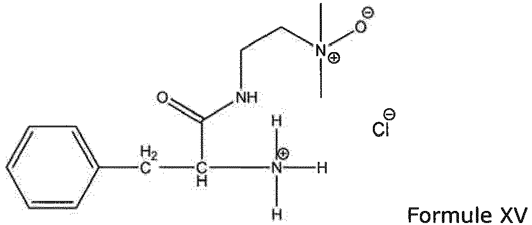

Dans un mode de réalisation de l'utilisation ou du procédé, le composé de formule I est le racémique du sel dichlorhydrate d'alpha-amino-N-[2-(N-hydroxy-N,N-diméthylaminium)éthyl] de benzene propanamide.

Dans un mode de réalisation de l'utilisation ou du procédé, le composé de formule I est le sel dichlorhydrate d'alpha-amino-N-[2-(N-hydroxy-N,N-diméthylaminium)éthyl] de benzene propanamide.

Dans un mode de réalisation de l'utilisation ou du procédé, le composé de formule I est le sel dichlorhydrate d'alpha-amino-N-[2-(N-hydroxy-N,N-diméthylaminium)éthyl] de benzene propanamide.

Dans un mode de réalisation de l'utilisation ou du procédé, le composé de formule I est le sel chlorhydrate de la 2-phényléthylamine et R₁ = R₂ = R₃ = H, L est une liaison, G = C₆H₅, m = n = 1, R₄ = R₅ = R₆ = R₇ = H et X⁻ = Cl⁻.

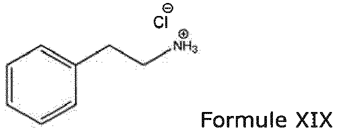

Dans un mode de réalisation de l'utilisation ou du procédé, le composé de formule I est le chlorure de benzyltriéthylammonium et R₁ = R₂ = R₃ = CH₃, L est une liaison, G = C₆H₅, m = 0, n = 1, R₄ = R₅ = H et X⁻ = Cl⁻.

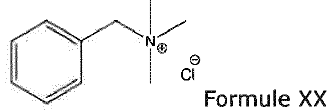

Dans un mode de réalisation de l'utilisation ou du procédé, le composé de formule I est le sel chlorhydrate de (2S-2-amino-(3-phénylpropanoyl)amino)éthanol et R₁ = R₂ = R₃ = H, L est une liaison, G = C₆H₅, m = 1, n = 1, R₄ = CONHCH₂CH₂OH, R₅ = H et X⁻ = Cl⁻.

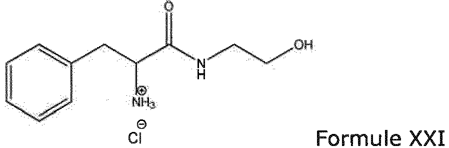

Dans un mode de réalisation de l'utilisation ou du procédé, le composé de formule I est le racémique du sel chlorhydrate de (2S-2-amino-(3-phénylpropanoyl)amino)éthanol.

Dans un mode de réalisation de l'utilisation ou du procédé, le composé de formule I est le sel chlorhydrate de (2S-2-amino-(3-phénylpropanoyl)amino)éthanol.

Dans un mode de réalisation de l'utilisation ou du procédé, le composé de formule I est le sel chlorhydrate de (2S-2-amino-(3-phénylpropanoyl)amino)éthanol.

Dans un mode de réalisation de l'utilisation ou du procédé, le composé de formule I est le sel chlorhydrate de la dopamine et R₁ = R₂ = R₃ = CH₃, L est une liaison, G = C₆H₅, m = 1, n = 1, R₄ = R₅ = R₆ = R₇ = H et X⁻ = Cl⁻.

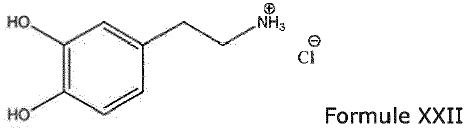

Dans un mode de réalisation de l'utilisation ou du procédé, le composé de formule I est le sel chlorhydrate de la 2-phénoxyéthylamine et R₁ = R₂ = R₃ = CH₃, L = O, G = C₆H₅, m = 1, n = 1, R₄ = R₅ = R₆ = R₇ = H et X⁻ = Cl⁻.

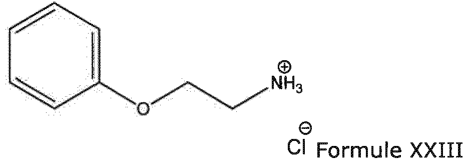

La présente invention concerne également un composé réducteur de viscosité de Formule XIV : 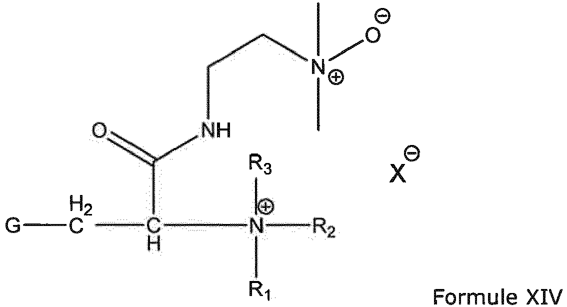 dans laquelle :
▪ G est un groupe phényle ou alkylphényle en C6 à C10, le groupe phényle pouvant être substitué par un ou plusieurs groupements hydroxyles,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ X⁻ est au moins un anion choisi dans le groupe constitué des halogènures, des carboxylates, des sulfates, phosphates et sulfonates,
▪ Les atomes de carbone asymétriques présentent une configuration R ou S.

La présente invention concerne également un composé réducteur de viscosité de Formule XV : 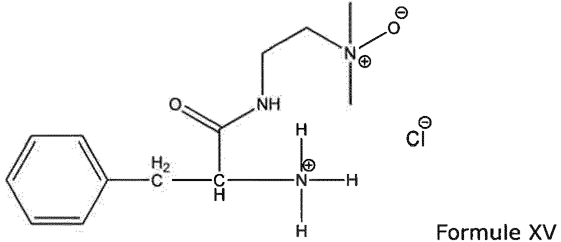 dans laquelle :
▪ Les atomes de carbone asymétriques présentent une configuration R ou S.

La présente invention concerne également une composition aqueuse (à viscosité reduite) comprenant un composé de formule XIV et une protéine comportant au moins un fragment d'anticorps dont la concentration est comprise en 50 et 350 mg/mL et dont le pH est compris entre 5 et 8.

Dans un mode de réalisation de la composition, la concentration du composé de formule XIV est comprise entre 10 et 200 mM.

Dans un mode de réalisation de la composition, la concentration du composé de formule XIV est comprise entre 25 et 200 mM.

Dans un mode de réalisation de la composition, la concentration du composé de formule XIV est comprise entre 50 et 200 mM.

Dans un mode de réalisation de la composition, la concentration du composé de formule XIV est comprise entre 50 et 175 mM.

Dans un mode de réalisation de la composition, la concentration du composé de formule XIV est comprise entre 100 et 250 mM.

Dans un mode de réalisation de la composition, la concentration du composé de formule XIV est comprise entre 100 et 200 mM.

Dans un mode de réalisation de la composition, la concentration du composé de formule XIV est comprise entre 100 et 175 mM.

La présente invention concerne également une composition aqueuse (à viscosité reduite) comprenant un composé de formule XV à une concentration comprise entre 10 et 250 mM et une protéine comportant au moins un fragment d'anticorps dont la concentration est comprise en 50 et 350 mg/mL et dont le pH est compris entre 5 et 8.

Dans un mode de réalisation de la composition, la concentration du composé de formule XV est comprise entre 10 et 200 mM.

Dans un mode de réalisation de la composition, la concentration du composé de formule XV est comprise entre 25 et 200 mM.

Dans un mode de réalisation de la composition, la concentration du composé de formule XV est comprise entre 50 et 200 mM.

Dans un mode de réalisation de la composition, la concentration du composé de formule XV est comprise entre 50 et 175 mM.

Dans un mode de réalisation de la composition, la concentration du composé de formule XV est comprise entre 100 et 250 mM.

Dans un mode de réalisation de la composition, la concentration du composé de formule XV est comprise entre 100 et 200 mM.

Dans un mode de réalisation de la composition, la concentration du composé de formule XV est comprise entre 100 et 175 mM.

Dans un mode de réalisation de la composition la concentration en protéine comportant au moins un fragment d'anticorps est comprise entre 100 et 350 mg/mL.

Dans un mode de réalisation de la composition la concentration en protéine comportant au moins un fragment d'anticorps est comprise entre 100 et 300 mg/mL.

Dans un mode de réalisation de la composition la concentration en protéine comportant au moins un fragment d'anticorps est comprise entre 100 et 250 mg/mL.

Dans un mode de réalisation de la composition la concentration en protéine comportant au moins un fragment d'anticorps est comprise entre 150 et 300 mg/mL.

Dans un mode de réalisation de la composition la concentration en protéine comportant au moins un fragment d'anticorps est comprise entre 150 et 250 mg/mL.

L'invention est illustrée par les exemples suivants, qui illustrent l'effet technique et la comparaison avec les composés de l'art antérieur.

### Exemple 1 : Préparation de solutions mères

Dans le tableau suivant les composés qui sont cités dans la suite de la partie expérimentale sont décrits et identifiés par un code RV X ou un nom abrégé Arg ou un nom NaCl.

| Code | Nom | Structure | Fournisseur Référence | Numéro CAS |
|---|---|---|---|---|
| Arg | sel chlorhydrate de la L-arginine | 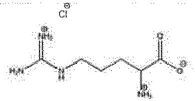 | Sigma 11039 | CAS# 1119-34-2 |
| RV 1 | N/A | N/A | N/A | N/A |
| RV 2 | sel chlorhydrate de (2S-2-amino-(3-phénylpropanoyl)amino)éth anol | 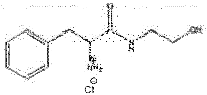 | Adocia | N/A |
| RV 3 | sel chlorhydrate de l'ester méthylique de la L-phénylalanine | 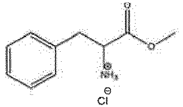 | Bachem E-2270 | CAS# 7524-50-7 |
| RV 4 | sel chlorhydrate du (S)-(-)-2-amino-3-phényl-1-propanol | 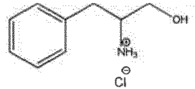 | Aldrich 190438 | CAS# 3182-95-4 |
| RV 5 | chlorure de benzyltriméthylammonium | 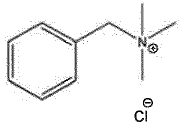 | Aldrich 228982 | CAS# 56-93-9 |
| RV 6 | sel chlorhydrate de la 2-phényléthylamine | 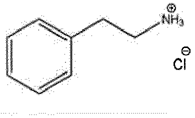 | Aldrich 241008 | CAS# 64-04-0 |
| RV 7 | sel chlorhydrate de la dopamine | 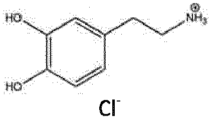 | Sigma H8502 | CAS# 62-31-7 |
| RV 8 | sel chlorhydrate de la 2-phénoxyéthylamine | 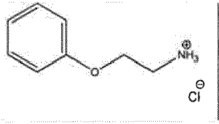 | Aldrich 448400 | CAS# 1758-46-9 |
| RV 9 | sel chlorhydrate de la L-phénylalaninamide | 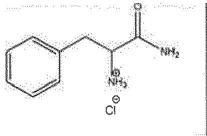 | Bachem E-2235 | CAS# 5241-58-7 |
| RV 9a | sel acétate de la L-phénylalaninamide | 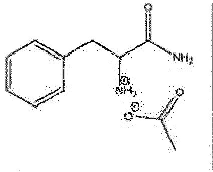 | Adocia | N/A |
| RV 9b | sel sulfate de la L-phénylalaninamide | 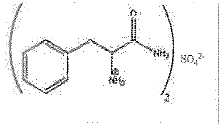 | Adocia | N/A |
| RV 9c | sel phosphate de la L-phénylalaninamide | 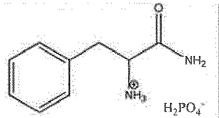 | Adocia | N/A |
| NaCl | chlorure de sodium | Na⁺Cl⁻ | Cooper 171 0500 | CAS# 7647-14-5 |
| RV 10 | N/A | N/A | N/A | N/A |
| RV 11 | sel chlorhydrate d'alpha-amino-N-[2-(N,N-diméthylamine-N-oxyde)éthyl] de benzène propanamide | 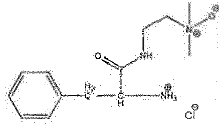 | Adocia | N/A |

| | | | | |
|---|---|---|---|---|
| N/A signifie non applicable. Arg : Solution de sel chlorhydrate de la L-arginine | | | | |

Le sel chlorhydrate de la L-arginine a été obtenu chez Sigma-Aldrich (Ref Sigma 11039, CAS# 1119-34-2) et a été solubilisé dans l'eau Milli-Q^{®} de sorte à obtenir une solution à 994 mM en sel chlorhydrate de la L-arginine.

### RV 2 : Solution de sel chlorhydrate de (2S-2-amino-(3-phénylpropanoyl)amino)éthanol

A 2,4 g (40 mmol) d'éthanolamine dissous dans du dichlorométhane sont ajoutés 10 g (37,7 mmol) de N-tert-butoxycarbonyl-L-phénylalanine. Le mélange est refroidi à 0°C puis 11,2 g (87 mmol) de diisopropylamine et 5,1 g (37,7 mmol) de hydroxybenzotriazole hydraté sont ajoutés. 9,4 g (49 mmol) de chlorhydrate de N-(3-diméthylaminopropyl)-N'-éthylcarbodiimide sont ensuite ajoutés et le mélange agité à température ambiante pendant 70 heures. Le milieu réactionnel est lavé avec une solution de NH₄Cl demi-saturée, une solution de NaHCO₃ saturée, de l'eau puis après séchage avec MgSO₄, la phase organique est concentrée pour donner 11,6 g de ((2S-2-[(tert-butoxycarbonyl)amino]-3-phénylpropanoyl)amino)éthanol.

A 10,5 g (34 mmol) de ((2S-2-[(tert-butoxycarbonyl)amino]-3-phénylpropanoyl)amino)éthanol dans le dichlorométhane à 0°C sont progressivement ajoutés 85 mL d'une solution de HCl (4M) dans le dioxane. Après une nuit à température ambiante, le milieu réactionnel est concentré et le solide obtenu lavé avec du diisopropyléther puis séché sous vide. 7,3 g de sel chlorhydrate de (2S-2-amino-(3-phénylpropanoyl)amino)éthanol sont obtenus.

Le produit est recristallisé dans l'éthanol pour donner un solide blanc.

Rendement : 3,8 g (43%)

RMN ¹H (DMSO-d₆, ppm) : 3,00-3,20 (4H) ; 3,25-3,40 (2H) ; 3,99 (1H) ; 4, 81 (1H) ; 7,25-7,35 (5H) ; 8,38 (3H) ; 8,66 (1H).

LC/MS (ESI): 207,1 ([M-Cl-H]⁻) ; ([M-Cl-H]⁻ calculé: 208,2).

Ce sel a ensuite été solubilisé dans l'eau Milli-Q^{®} de sorte à obtenir une solution à 1019 mM en sel chlorhydrate de (2S-2-amino-(3-phénylpropanoyl)amino)éthanol.

### RV 3 : Solution de sel chlorhydrate de l'ester méthylique de la L-phénylalanine

Le sel chlorhydrate de l'ester méthylique de la L-phénylalanine (RV 3) a été obtenu chez Bachem (Ref E-2270, CAS# 7524-50-7) et a été solubilisé dans l'eau Milli-Q^{®} de sorte à obtenir une solution à 998 mM en sel chlorhydrate de l'ester méthylique de la L-phénylalanine.

### RV 4 : Solution de sel chlorhydrate du (S)-(-)-2-amino-3-phényl-1-propanol

Le sel chlorhydrate du (S)-(-)-2-amino-3-phényl-1-propanol (RV 4) a été obtenu en solubilisant le (S)-(-)-2-amino-3-phényl-1-propanol obtenu chez Sigma-Aldrich (Ref Aldrich 190438, CAS# 3182-95-4) dans l'eau Milli-Q^{®} et en ajoutant de l'acide chlorhydrique (HCl) de sorte à obtenir une solution à 992 mM en sel chlorhydrate du (S)-(-)-2-amino-3-phényl-1-propanol.

### RV 5 : chlorure de benzyltriméthylammonium

Le chlorure de benzyltriméthylammonium (RV 5) a été obtenu chez Sigma-Aldrich (Ref Aldrich 228982, CAS# 56-93-9) et a été solubilisé dans l'eau Milli-Q^{®} de sorte à obtenir une solution à 1030 mM en chlorure de benzyltriméthylammonium.

### RV 6 : Solution de sel chlorhydrate de la 2-phényléthylamine

Le sel chlorhydrate de la 2-phényléthylamine (RV 6) a été obtenu en solubilisant la 2-phényléthylamine obtenue chez Sigma-Aldrich (Ref Sigma 241008, CAS# 64-04-0) dans l'eau Milli-Q^{®} et en ajoutant de l'acide chlorhydrique de sorte à obtenir une solution à 881 mM en sel chlorhydrate de la 2-phényléthylamine.

### RV 7 : Solution de sel chlorhydrate de la dopamine

Le sel chlorhydrate de la dopamine (RV 7) a été obtenu chez Sigma-Aldrich (Ref Sigma H8502, CAS# 62-31-7) et a été solubilisé dans l'eau Milli-Q^{®} de sorte à obtenir une solution à 1071 mM en sel chlorhydrate de la dopamine.

### RV8 : Solution de sel chlorhydrate de la 2-phénoxyéthylamine

Le sel chlorhydrate de la 2-phénoxyéthylamine (RV 8) a été obtenu en solubilisant la 2-phénoxyéthylamine obtenue chez Sigma-Aldrich (Ref Aldrich 448400, CAS# 1758-46-9) dans l'acide chlorhydrique de sorte à obtenir une solution à 826 mM en sel chlorhydrate de 2-phénoxyéthylamine.

### RV 9 : Solution de sel chlorhydrate de la L-phénylalaninamide

Le sel chlorhydrate de la L-phénylalaninamide (RV 9) a été obtenu en solubilisant la L-phénylalaninamide obtenue chez Bachem (Ref E-2235, CAS# 5241-58-7) dans l'eau Milli-Q^{®} et en ajoutant de l'acide chlorhydrique de sorte à obtenir une solution à 955 mM en sel chlorhydrate de la L-phénylalaninamide.

### RV 9a : Solution de sel acétate de la L-phénylalaninamide

Le sel acétate de la L-phénylalaninamide (RV 9a) a été obtenu en solubilisant la L-phénylalaninamide obtenue chez Bachem (Ref E-2235, CAS# 5241-58-7) dans l'eau Milli-Q^{®} et en ajoutant de l'acide acétique de sorte à obtenir une solution à 1196 mM en sel acétate de la L-phénylalaninamide.

### RV 9b : Solution de sel sulfate de la L-phénylalaninamide

Le sel sulfate de la L-phénylalaninamide (RV 9b) a été obtenu en solubilisant la L-phénylalaninamide obtenue chez Bachem (Ref E-2235, CAS# 5241-58-7) dans l'eau Milli-Q^{®} et en ajoutant de l'acide sulfurique de sorte à obtenir une solution à 1250 mM en sel sulfate de la L-phénylalaninamide.

### RV 9c : Solution de sel phosphate de la L-phénylalaninamide

Le sel phosphate de la L-phénylalaninamide (RV 9c) a été obtenu en solubilisant la L-phénylalaninamide obtenue chez Bachem (Ref E-2235, CAS# 5241-58-7) dans l'eau Milli-Q^{®} et en ajoutant de l'acide phosphorique de sorte à obtenir une solution à 1250 mM en sel phosphate de la L-phénylalaninamide.

### RV 11 : Solution de sel chlorhydrate d'alpha-amino-N-[2-(N,N-diméthylamine-N-oxyde)éthyl] de benzène propanamide

A une solution de 21,7 g (113 mmol) de chlorhydrate de N-(3-diméthylaminopropyl)-N'-éthylcarbodiimide, 17,3 g (113 mmol) de 1-hydroxybenzotriazole et de 20 g (75,4 mmol) de N-tert-butoxycarbonyl-L-phénylalanine dans le DMF sont ajoutés à température ambiante 10 g (113 mmol) de N,N'-diméthyl-éthylène-1,2-diamine et 15,25 g (150,8 mmol) de N-méthylmorpholine. Le mélange est agité à température ambiante pendant 24 h, concentré sous pression réduite et extrait avec de l'acétate d'éthyle. La phase organique est lavée avec une solution saturée de NaHCO₃ puis séchée sur Na₂SO₄, filtrée et concentrée sous pression réduite pour donner un solide jaune. Par recristallisation à l'aide d'acétate d'éthyle, le [(1S)-2-[[2-diméthylamino)éthyl]amino]-2-oxo-1-(phénylméthyl)éthyl]-ester d'acide carbamique est obtenu sous la forme d'un solide blanc.

Rendement : 12,9 g (51%).

RMN ¹H (DMSO-d₆, ppm) : 1,2-1,3 (9H) ; 2,12 (6H), 2,21-2,25 (2H), 2,68-2,72 (1H), 2,85-2,90 (1H), 3,05-3,20 (2H), 4,05-4,15 (1H), 6,89-6,92 (1H), 7,10-7,25 (5H), 7,73 (1H).

LC/MS (ESI): 336,6 ([M+H]⁺) ; ([M+H]⁺ calculé: 335,5).

Sous atmosphère inerte et à 0°C, à une solution de 12,9 g (38,45 mmol) de [(1S)-2-[[2-diméthylamino)éthyl]amino]-2-oxo-1-(phénylméthyl)éthyl]-ester d'acide carbamique dans le chloroforme (77 mL) est ajoutée une solution d'acide métachloroperbenzoïque (13,5 g) dans du chloroforme (88 mL). Après 10 min de réaction, le milieu est versé dans une solution saturée de Na₂CO₃ et la phase aqueuse est extraite trois fois avec du chloroforme. Les phases organiques combinées sont lavées avec une solution de NaCl saturée puis séchées sur Na₂SO₄. Le pH de la phase aqueuse est montée à 12 et extraite 3 fois avec de l'acétate d'éthyle. Les phases organiques sont combinées, séchées sur Na₂SO₄ et après concentration, un solide blanc est obtenu. Ce solide est engagé tel quel dans l'étape suivante de déprotection de la fonction amine primaire. Sous atmosphère inerte et à 0°C, à une solution du solide blanc (10,8 g) dans le dichlorométhane (150 mL) sont ajoutés 54 mL de HCl (4N) dans le dioxane. Après 3h d'agitation à 0°C, le milieu est extrait avec 3x100 mL d'une solution d'HCl (1N). Les phases aqueuses combinées sont lavées avec 60 mL de dichlorométhane, filtrées et concentrées sous pression réduite. L'huile jaune obtenue est reprise dans 50 mL d'éthanol absolue et le NaCl précipité est éliminé par filtration sur fritté. Le solide blanc obtenu après concentration sous pression réduite est purifié par recrystallisation dans l'éthanol pour donner des cristaux blanc. Le produit est repris dans 30 mL d'eau et lyophilisé.

Rendement : 5,7 g (57%).

RMN ¹H (DMSO-d₆, ppm) : 3,05-3,10 (2H), 3,35-3,75 (10H), 4,05 (1H), 7,25-7,35 (5H), 8,52 (3H) 9,19-9,22 (1H), 12,87 (1H).

LC/MS (ESI): 252,6 ([M-2Cl-H]⁻) ; ([M-2Cl-H]⁻ calculé: 253,2).

Le lyophilisat du sel dichlorhydrate d'alpha-amino-N-[2-(N-hydroxy-N,N-diméthylaminium)éthyl] de benzene propanamide ainsi obtenu est solubilisé dans l'eau Milli-Q^{®} et en ajoutant de l'hydroxyde de sodium de sorte à obtenir une solution à 1223 mM en dichlorhydrate d'alpha-amino-N-[2-(N-hydroxy-N,N-diméthylaminium)éthyl] de benzene propanamide.

### EXEMPLE 2 - Effet des RVs 2 et 9 comparativement à celui de Arg et NaCl sur la viscosité de différentes solutions de protéines thérapeutiques hautement concentrées.

Cet exemple illustre comment deux des RVs appartenant à la famille des RVs utilisés selon l'invention, les RV 2 et RV 9, réduisent la viscosité de trois formulations d'anticorps monoclonaux et d'une formulation de protéine de fusion en comparaison des composés de référence tels que Arg et NaCl.

Pour cet exemple, trois solutions d'anticorps monoclonaux i.e. une solution de bevacizumab (un IgG1 commercialisé sous le nom d'Avastin^{®} par Genentech / Hoffmann La Roche), une solution d'infliximab (un IgG1/κ commercialisé sous le nom de Remicade^{®} par Schering-Plough), une solution de rituximab (un IgG1/κ commercialisé sous le nom de mAbThera^{®} par Hoffmann La Roche) et une solution de protéine de fusion i.e. une solution d'abatacept (IgG1-CTLA4 commercialisé sous le nom de Orencia^{®} par Bristol-Meyer Squibb), ont été concentrées par ultrafiltration sur des unités de filtration Amicon^{®} Ultra-15 possédant un seuil de coupure de 50 kDa.

La concentration en protéine de chaque solution concentrée a été mesurée par spectroscopie UV (absorbance à 280 nm) après dilution gravimétrique des échantillons.

Les solutions de rituximab (10mg/mL) et de bevacizumab (25 mg/mL) ont été concentrées respectivement à 320 mg/mL et 240 mg/mL.

Infliximab et abatacept , commercialisés sous forme de lyophilisats, ont été reconstitués à l'aide d'eau pour injection à la concentration suggérée par le fabricant (i.e. 10 mg/mL pour infliximab et 25 mg/mL pour abatacept) puis concentrées à respectivement 199 mg/mL et 280 mg/mL.

Les pH des formulations commerciales des protéines thérapeutiques utilisées pour ces essais sont résumés dans le tableau ci-dessous :

| | rituximab | bevacizumab | infliximab | abatacept |
|---|---|---|---|---|
| pH de la formulation commerciale | 6,5 ± 0,2 | 6,2 ± 0,2 | 7,2 ± 0,2 | 7,5 ± 0,5 |

L'effet des RVs 2 et 9 sur la viscosité de ces quatre solutions de protéines thérapeutiques hautement concentrées a ensuite été évalué comparativement à celui de Arg et NaCl. Pour cela, la viscosité a été mesurée à l'aide d'un rhéomètre cône/plan (TA Instrument, AR 2000 Ex, géométrie de diamètre 20 mm, cône de 0,5131°, trappe à solvent remplie d'eau) à une température de 21°C et en mode Balayage (Flow Sweep) avec des taux de cisaillements compris entre 5000 et 50 s⁻¹ (3 points par décade - steady-state sensing).

Pour cet exemple, les formulations « Protéine + RV », « Protéine + Arg » et « Protéine + NaCl » ont été préparées par mélange d'unedes solutions de RV, Arg ou NaCl préparées à l'exemple 1 et une des solutions de protéine concentrée préparées précédemment de sorte à atteindre une concentration finale en RV, Arg ou NaCl de 150 mM et une concentration finale en protéine de 250 mg/mL pour bevacizumab, 196 mg/mL pour rituximab, 230 mg/mL pour abataceptet 107 mg/mL pour infliximab. Les échantillons ainsi préparés ont été stockés à température ambiante et ont été analysés à l'aide du rhéomètre dans les 24 heures suivant leur préparation.

Les solutions témoins sont préparées par dilution dans l'eau Milli-Q^{®}, de sorte à atteindre une concentration finale en protéine de 250 mg/mL pour bevacizumab, 196 mg/mL pour rituximab, 230 mg/mL pour abataceptet 107 mg/mL pour infliximab. Le pH de ces formulations est le même que celui de la formulation commerciale de la protéine thérapeutique correspondante.

Les résultats des mesures relatives à ce deuxième exemple sont présentés dans le tableau I ci-après sous la forme de pourcentages de réduction de viscosité par rapport à la solution témoin de protéine thérapeutique seule, à la même concentration en protéine que les formulations « Protéine + RV » ou « Protéine + Arg » ou « Protéine + NaCl ».

**Tableau I**

| Concentration testée | % de réduction de viscosité par rapport au contrôle « Protéine Seule » | | | |
|---|---|---|---|---|
| | bevacizumab 250 mg/mL | rituximab 196 mg/mL | abatacept 230 mg/mL | infliximab 107 mg/mL |
| 150 mM NaCl | ≤0% | 21% | 7% | ≤0% |
| 150 mM Arg | 26% | 58% | 31% | 32% |
| 150 mM RV 2 | 44% | 43% | 27% | 56% |
| 150 mM RV 9 | 35% | 48% | 42% | 52% |

Les données présentées dans le tableau I démontrent qu'en présence de 150 mM de RV 2 ou de RV 9, la viscosité des 4 solutions de protéines hautement concentrées est significativement réduite.

### EXEMPLE 3 - Effet de différents RVs sur la viscosité de deux formulations aqueuses d'anticorps monoclonaux hautement concentrées comparativement à celui de Arg et NaCl.

Cet exemple illustre comment différents RVs utilisés selon l'invention abaissent la viscosité de solutions hautement concentrées d' infliximab et de rituximab en comparaison de référence tels que Arg et NaCl.

Dans cette étude, les solutions d'infliximab et de rituximabhautement concentrées utilisées sont les mêmes que celles qui ont été préparées et utilisées précédemment. Les formulations « Protéine + RV » ont été préparées en présence de 150 mM de différents RVs utilisés selon l'invention. La viscosité de ces formulations a ensuite été mesurée comme décrit à l'exemple 2 et comparée à celle obtenue en présence de Arg à 150 mM. Le pH de ces formulations est le même que celui de la formulation de protéine thérapeutique correspondante, c'est-à-dire 7,2 ± 0,2 pour infliximab, et 6,5 ± 0,2 pour rituximab.

Les résultats des mesures relatives à ce troisième exemple sont présentés dans les tableaux II et III ci-après sous la forme de pourcentages de réduction de viscosité par rapport à la solution témoin de protéine thérapeutique seule, à la même concentration en protéine que les formulations « Protéine + RV » ou « Protéine + Arg » ou « Protéine + NaCl ».

**Tableau II**

| | Concentration testée | % de réduction de viscosité par rapport au contrôle « Protéine Seule » |
|---|---|---|
| infliximab 107 mg/mL | 150 mM NaCl | ≤0% |
| | 150 mM Arg | 32% |
| | 150 mM RV 4 | 36% |
| | 150 mM RV 6 | 38% |
| | 150 mM RV 7 | 38% |
| | 150 mM RV 8 | 46% |
| | 150 mM RV 5 | 49% |
| | 150 mM RV 9 | 52% |
| | 150 mM RV 2 | 56% |
| | 150 mM RV 3 | 64% |

Les données présentées dans le tableau II démontrent qu'utilisées à 150 mM, tous les RVs utilisés selon l'invention réduisent la viscosité de infliximab à 107 mg/mL de façon significative.

**Tableau III**

| | Concentration testée | % de réduction de viscosité par rapport au contrôle « Protéine Seule » |
|---|---|---|
| rituximab 196 mg/mL | 150 mM NaCl | 21% |
| | 150 mM Arg | 58% |
| | 150 mM RV 3 | 28% |
| | 150 mM RV 4 | 37% |
| | 150 mM RV 5 | 38% |
| | 150 mM RV 6 | 39% |
| | 150 mM RV 7 | 39% |
| | 150 mM RV 2 | 43% |
| | 150 mM RV 9 | 48% |
| | 150 mM RV 8 | 52% |

Les données présentées dans le tableau III démontrent qu'utilisées à 150 mM, tous les RVs utilisés selon l'invention réduisent la viscosité de rituximab à 196 mg/mL de façon significative.

### EXEMPLE 4 - Evaluation de l'effet de la concentration en RV 9 sur la diminution de viscosité d'une formulation aqueuse d'anticorps monoclonal hautement concentrée comparativement à celui de Arg.

La solution d'infliximab hautement concentrée utilisée est la même que celle qui a été préparée et utilisée précédemment. Les formulations « Protéine + RV » ont été préparées en présence de concentrations croissantes de 0 à 200 mM d'Arg ou RV 9. Le pH de ces formulations est le même que celui de la formulation commerciale d'infliximab, c'est-à-dire 7,2 ± 0,2.

La viscosité des formulations ainsi préparées a été mesurée comme décrit dans l'exemple 2.

Les résultats des mesures relatives à ce quatrième exemple sont présentés dans le tableau IV ci-après sous la forme de pourcentages de réduction de viscosité par rapport à la solution témoin de protéine thérapeutique seule, à la même concentration en protéine que les formulations « Protéine + RV ».

**Tableau IV**

| | Concentration testée | Arg % de réduction de viscosité par rapport au contrôle « Protéine Seule » | RV 9 % de réduction de viscosité par rapport au contrôle « Protéine Seule » |
|---|---|---|---|
| infliximab 107 mg/mL | 25 mM | 9% | 19% |
| | 50 mM | 13% | 31% |
| | 100 mM | 9% | 41% |
| | 150 mM | 31% | 44% |
| | 200 mM | 34% | 56% |

Les données présentées dans le tableau IV montrent qu'à 150 mM, RV 9 est déjà 2 fois plus efficace qu'Arg. A 100 mM, RV 9 est environ 4 fois plus efficace qu'Arg. Par ailleurs, ces résultats montrent aussi qu'il suffit de 50 mM de RV 9 pour obtenir une réduction de viscosité équivalente à celle obtenue à l'aide d'Arg à 150 mM.

### EXEMPLE 5 - Evaluation de l'effet de la concentration d'un anticorps monoclonal sur l'abaissement de viscosité en présence des RVs utilisés selon l'invention.

L'objectif de cet exemple est de comparer l'efficacité pour la réduction de la viscosité de RV 9 à celle de Arg, en fonction de la concentration en anticorps.

La solution d'infliximab hautement concentrée utilisée est la même que celle qui a été préparée et utilisée précédemment. Les formulations « Protéine + RV » ont été préparées en présence de 150 mM d'Arg ou RV 9 et de concentrations croissantes de 80 mg/mL à 110 mg/mL en infliximab.

Le pH de ces formulations est le même que celui de la formulation commerciale d'infliximab, c'est-à-dire 7,2 ± 0,2.

La viscosité des formulations ainsi préparées a été mesurée comme décrit dans l'exemple 2.

Les résultats des mesures relatives à ce cinquième exemple sont présentés dans le tableau V ci-après sous la forme de pourcentages de réduction de viscosité par rapport à la solution infliximab témoin.

**Tableau V**

| | % de réduction de viscosité par rapport à la solution infliximab témoin | |
|---|---|---|
| Concentration en infliximab (mg/mL) | Arg à 150 mM | RV 9 à 150 mM |
| 80 | 25% | 50% |
| 90 | 30% | 52% |
| 100 | 39% | 55% |
| 110 | 22% | 49% |

Les données présentées dans le tableau V montrent que quelle que soit la concentration d'infliximab, Arg n'abaisse la viscosité de la solution que d'environ 29% en moyenne tandis que RV 9 l'abaisse d'environ 52% en moyenne.

### EXEMPLE 6 - Evaluation de l'effet du contre-ion d'un RV utilisé selon l'invention sur l'abaissement de viscosité d'une formulation aqueuse d'anticorps monoclonal hautement concentrée apporté par ce RV.

L'objectif de cet exemple est d'évaluer l'impact du contre-ion (contre-anion en l'occurrence) sur l'abaissement de viscosité d'une solution d'infliximab à 115 mg/mL en présence de RV 9 modifié, où HCl a été remplacé par différents contre ions. Les contre-anions testés sont : chlore (RV 9), acétate (RV 9a), phosphate (RV 9b), et sulfate (RV 9c).

La solution d'infliximab hautement concentrée utilisée a été préparée de la même façon que celle utilisée précédemment. Les formulations « Protéine + RV » ont été préparées par addition d'une solution de RV (9, 9a, 9b ou 9c) préparée à l'exemple 2 (à 1,2 M environ) et d'eau Milli-Q^{®} à la solution d'infliximab concentrée de sorte à atteindre une concentration finale en RV de 150 mM et une concentration finale en protéine de 115 mg/mL. Le pH de ces formulations est le même que celui de la formulation commerciale d'infliximab, c'est-à-dire 7,2 ± 0,2. La viscosité des formulations ainsi préparées a été mesurée comme décrit dans l'exemple 2. Les résultats des mesures relatives à ce sixième exemple sont présentés dans le tableau VI ci-après sous la forme de pourcentages de réduction de viscosité par rapport à la solution témoin de protéine thérapeutique seule, à la même concentration en protéine.

**Tableau VI**

| | Concentration testée | % de réduction de viscosité par rapport au contrôle « Protéine Seule » |
|---|---|---|
| infliximab 115 mg/mL | 150 mM RV 9 | 64% |
| | 150 mM RV 9a | 54% |
| | 150 mM RV 9b | 54% |
| | 150 mM RV 9c | 56% |

Les données présentées dans le tableau VI montrent que RV9, RV9a, RV9b et RV9c réduisent tous les 5 d'au moins 54% la viscosité d'infliximab à 115 mg/mL.

### EXEMPLE 7 - Effet des RVs 2 et 11 sur la viscosité de différentes formulations de protéines thérapeutiques hautement concentrées comparativement à celui de Arg et NaCl.

Cet exemple illustre comment le RV 2 et le RV 11 appartenant à la famille des RVs utilisés selon l'invention réduisent la viscosité de deux formulations d'anticorps monoclonaux en comparaison des composés de référence tels que Arg et NaCl.

Pour cet exemple, deux formulations d'anticorps monoclonaux i.e. infliximab et trastuzumab (un IgG1/κ commercialisé sous le nom d'Herceptin^{®} par Genentech) ont été concentrées par ultrafiltration sur des unités de filtration à centrifuger Amicon^{®} Ultra-50 possédant un seuil de coupure de 50 kDa. La concentration en protéine de chaque solution concentrée a été mesurée par spectroscopie UV (absorbance à 280 nm) après dilution gravimétrique des échantillons.

Les anticorps monoclonaux infliximab et trastuzumab, commercialisés sous forme de lyophilisats, ont été reconstitués à l'aide d'eau pour injection à la concentration suggérée par les fabricants (i.e. 10 mg/mL pour infliximab et 21 mg/mL pour trastuzumab). Une fois reconstituées, les formulations d'infliximab et de trastuzumab ont été concentrées à respectivement 212 mg/mL et 277 mg/mL.

Le pH des formulations commerciales de protéines thérapeutiques utilisées pour ces essais est indiqué dans le tableau ci-dessous :

| | infliximab | trastuzumab |
|---|---|---|
| pH de la formulation commerciale | 7,2 ± 0,2 | 6 ± 0,2 |

L'effet des RVs 2 et 11 sur la viscosité de ces deux formulations de protéines thérapeutiques hautement concentrées a ensuite été évalué comparativement à celui de Arg et NaCl. Pour cela, la viscosité a été mesurée à l'aide d'un rhéomètre cône/plan (TA Instrument, AR 2000 Ex, géométrie de diamètre 20 mm, cône de 0,5131°, trappe à solvent remplie d'eau) à une température de 25°C et en mode Balayage (Flow Sweep) avec des taux de cisaillements compris entre 5000 et 50 s⁻¹ (3 points par décade - steady-state sensing).

Pour cet exemple, les formulations « Protéine + RV » ont été préparées par mélange d'une solution de RV à environ 1 M obtenues à l'exemple 1 avec une des formulations d'infliximab et de trastuzumab concentrées précédemment préparées et le tampon correspondant de sorte à atteindre une concentration finale en RV de 150 mM et une concentration finale en protéine de 125 mg/mL pour infliximab et 200 mg/mL pour trastuzumab. Les échantillons ainsi préparés ont été stockés à température ambiante et ont été analysés à l'aide du rhéomètre dans les 72 heures suivant leur préparation.

Les solutions témoins sont préparées par dilution dans l'eau Milli-Q^{®}, de sorte à atteindre une concentration finale en protéine de 125 mg/mL pour infliximab et 200 mg/mL pour trastuzumab. Le pH de ces formulations est le même que celui de la formulation commerciale de la protéine thérapeutique correspondante.

Les résultats des mesures relatives à ce deuxième exemple sont présentés dans le tableau VII ci-après sous la forme de pourcentages de réduction de viscosité par rapport à la solution témoin de protéine thérapeutique seule, à la même concentration en protéine que les formulations « Protéine + RV ».

**Tableau VII**

| Concentration testée | % de réduction de viscosité par rapport au contrôle « Protéine Seule » | |
|---|---|---|
| | infliximab | trastuzumab |
| | 125 mg/mL | 200mg/mL |
| 150 mM NaCl | ≤0% | 38% |
| 150 mM Arg | 11% | 56% |
| 150 mM RV 2 | 44% | 63% |
| 150 mM RV 11 | 42% | 52% |

Les données présentées dans le tableau VII démontrent qu'en présence de 150 mM de RV 2 ou 11, la viscosité des 2 solutions de protéines hautement concentrées est significativement réduite.

### EXEMPLE 8 : Exemples de compositions selon l'invention.

**8.1 Composition sous forme de solution hautement concentrée d'infliximab dont le pH est de 7,2 comprenant du RV 11 .**

| Composition 1 | |
|---|---|
| Ingrédient : | Concentration : |
| Infliximab | 125 mg/mL |
| Phosphate de sodium | 30 mM |
| Sucrose | 86 mM |
| Polysorbate 80 | 474 µM |
| sel chlorhydrate d'alpha-amino-N-[2-(N,N-diméthylamine-N-oxyde)éthyl] de benzène propanamide (RV 11) | 150 mM |
| Eau pour injection, USP | qsp |

**8.2 Composition sous forme de solution hautement concentrée de trastuzumab dont le pH est de 6,0 comprenant du RV 11**

| Composition 2 | |
|---|---|
| Ingrédient : | Concentration: |
| Trastuzumab | 200 mg/mL |
| L-Histidine | 1,7 mM |
| Dihydrate d'α,α-trehalose | 38 mM |
| Polysorbate 20 | 653 µM |
| sel chlorhydrate d'alpha-amino-N-[2-(N,N-diméthylamine-N-oxyde)éthyl] de benzène propanamide (RV 11) | 150 mM |
| Eau pour injection, USP | qsp- |

**8.3 Composition sous forme de solution hautement concentrée de trastuzumab dont le pH est de 6,0 comprenant du RV 2**

| Composition 3 | |
|---|---|
| Ingrédient : | Concentration : |
| Trastuzumab | 200 mg/mL |
| L-Histidine | 1,7 mM |
| Dihydrate d'α,α-trehalose | 38 mM |
| Polysorbate 20 | 675 µM |
| sel chlorhydrate de (2S-2-amino-(3-phénylpropanoyl)amino)éthanol (RV 2) | 150 mM |
| Eau pour injection, USP | qsp- |

**8.4 Composition sous forme de solution hautement concentrée d'infliximab dont le pH est de 7,2 comprenant du RV 3**

| Composition 4 | |
|---|---|
| Ingrédient : | Concentration : |
| Infliximab | 107 mg/mL |
| Phosphate de sodium | 27 mM |
| Sucrose | 79 mM |
| Polysorbate 80 | 411 µM |
| Sel chlrorhydrate de l'ester méthylique de la L-phénylalanine (RV 3) | 150 mM |
| Eau pour injection, USP | qsp- |

**8.5 Composition sous forme de solution hautement concentrée de rituximab dont le pH est de 6,5 comprenant du RV 4**

| Composition 5 | |
|---|---|
| Ingrédient : | Concentration : |
| Rituximab | 196 mg/mL |
| Sodium citrate dihydrate | 15,33 mM |
| Sodium Chloride | 94 mM |
| Polysorbate 80 | 10,5 mM |
| sel chlorhydrate du (S)-(-)-2-amino-3-phényl-1-propanol (RV 4) | 150 mM |
| Eau pour injection, USP | qsp- |

**8.6 Composition sous forme de solution hautement concentrée d'infliximab dont le pH est de 7,2 comprenant du RV 5**

| Composition 6 | |
|---|---|
| Ingrédient : | Concentration : |
| Infliximab | 107 mg/mL |
| Phosphate de sodium | 27 mM |
| Sucrose | 79 mM |
| Polysorbate 80 | 411 µM |
| chlorure de benzyltriméthylammonium (RV 5) | 150 mM |
| Eau pour injection, USP | qsp- |

**8.7 Composition sous forme de solution hautement concentrée de rituximab dont le pH est de 6,5 comprenant du RV 6**

| Composition 7 | |
|---|---|
| Ingrédient : | Concentration : |
| Rituximab | 196 mg/mL |
| Sodium citrate dihydrate | 15,33 mM |
| Sodium Chloride | 94 mM |
| Polysorbate 80 | 10,5 mM |
| sel chlorhydrate de la 2-phényléthylamine (RV 6) | 150 mM |
| Eau pour injection, USP | qsp- |

**8.8 Composition sous forme de solution hautement concentrée de rituximab dont le pH est de 6,5 comprenant du RV 7**

| Composition 8 | |
|---|---|
| Ingrédient : | Concentration : |
| Rituximab | 196 mg/mL |
| Sodium citrate dihydrate | 15,33 mM |
| Sodium Chloride | 94 mM |
| Polysorbate 80 | 10,5 mM |
| sel chlorhydrate de la dopamine (RV 7) | 150 mM |
| Eau pour injection, USP | qsp- |

**8.9 Composition sous forme de solution hautement concentrée de rituximab dont le pH est de 6,5 comprenant du RV 8**

| Composition 9 | |
|---|---|
| Ingrédient : | Concentration : |
| Rituximab | 196 mg/mL |
| Sodium citrate dihydrate | 15,33 mM |
| Sodium Chloride | 94 mM |
| Polysorbate 80 | 10,5 mM |
| sel chlorhydrate de la 2-phénoxyéthylamine (RV 8) | 150 mM |
| Eau pour injection, USP | qsp- |

**8.10 Composition sous forme de solution hautement concentrée d'infliximab dont le pH est de 7,2 comprenant du RV 9**

| Composition 10 | |
|---|---|
| Ingrédient : | Concentration: |
| Infliximab | 115 mg/mL |
| Phosphate de sodium | 29 mM |
| Sucrose | 85 mM |
| Polysorbate 80 | 440 µM |
| sel chlorhydrate de la L-phénylalaninamide (RV 9) | 150 mM |
| Eau pour injection, USP | qsp- |

**8.11 Composition sous forme de solution hautement concentrée d'infliximab dont le pH est de 7,2 comprenant du RV 9a**

| Composition 11 | |
|---|---|
| Ingrédient : | Concentration: |
| Infliximab | 115 mg/mL |
| Phosphate de sodium | 29 mM |
| Sucrose | 85 mM |
| Polysorbate 80 | 440 µM |
| sel acétate de la L-phénylalaninamide (RV 9a) | 150 mM |
| Eau pour injection, USP | qsp- |

**8.12 Composition sous forme de solution hautement concentrée d'infliximab dont le pH est de 7,2 comprenant du RV 9b**

| Composition 12 | |
|---|---|
| Ingrédient : | Concentration: |
| Infliximab | 115 mg/mL |
| Phosphate de sodium | 29 mM |
| Sucrose | 85 mM |
| Polysorbate 80 | 440 µM |
| sel sulfate de la L-phénylalaninamide (RV 9b) | 150 mM |
| Eau pour injection, USP | qsp- |

**8.13 Composition sous forme de solution hautement concentrée d'infliximab dont le pH est de 7,2 comprenant du RV 9c**

| Composition 13 | |
|---|---|
| Ingrédient : | Concentration: |
| Infliximab | 115 mg/mL |
| Phosphate de sodium | 29 mM |
| Sucrose | 85 mM |
| Polysorbate 80 | 440 µM |
| sel phosphate de la L-phénylalaninamide (RV 9c) | 150 mM |
| Eau pour injection, USP | qsp- |

## Revendications

1. Utilisation d'un composé de formule I, à une concentration dans la formulation finale comprise entre 10 et 250 mM : 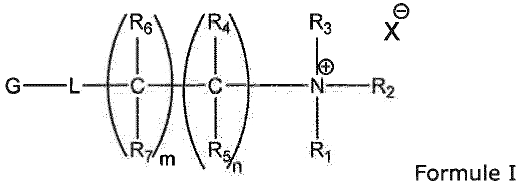 dans laquelle :
▪ G est un groupe phényle ou alkylphényle en C6 à C10, le groupe phényle pouvant être substitué par un ou plusieurs groupements hydroxyles,
▪ L est soit une liaison, soit une fontion choisie dans le groupe constitué de la fonction éther, de la fonction carbamate ou de la fonction amide,
▪ m et n sont des entiers identiques ou différents compris entre 0 et 2 tel que 0 < m + n ≤ 3,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ R₄, R₅, R₆, et R₇, identiques ou différents sont soit un atome d'hydrogène, soit une chaîne comprenant 1 à 6 atomes de carbone, mais ne porte pas de fonction acide carboxylique,
▪ X⁻ est un anion choisi dans le groupe constitué des halogènures, des carboxylates, des sulfates, phosphates et sulfonates,
▪ Les atomes de carbone asymétriques présentent une configuration R ou S.
ledit composé ayant une solubilité dans l'eau ≥ 20 mM à un pH compris entre 5 et 8, pour abaisser la viscosité d'une solution difficilement injectable d'au moins une protéine comportant au moins un fragment d'anticorps dont la concentration est comprise en 50 et 350 mg/mL et dont le pH est compris entre 5 et 8, d'une valeur d'au moins 15% par rapport à la viscosité d'une solution d'au moins une protéine comportant au moins un fragment d'anticorps de même concentration et de même pH ne contenant pas ledit composé.

2. Utilisation selon la revendication 1, **caractérisée en ce que** la protéine comportant au moins un fragment d'anticorps est un anticorps monoclonal.

3. Utilisation selon la revendication 1, **caractérisée en ce que** le composé est choisi parmi les composés de formule I dans laquelle au moins un groupe R₄, R₅, R₆ ou R₇ n'est pas un atome d'hydrogène et comprend une fonction amine-N-oxyde.

4. Utilisation selon l'une quelconque des revendications 1 à 3, **caractérisée en ce que** le composé est choisi parmi les composés de formule II : 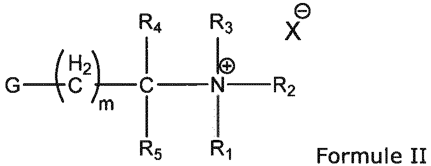 dans laquelle :
▪ G est un groupement phényle ou alkylphényle en C6 à C10, le groupe phényle pouvant être substitué par un ou plusieurs groupements hydroxyles,
▪ m est un entier égal à 0 ou 1,
R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₄ est une chaîne comprenant de 1 à 6 atomes de carbone, ladite chaine pouvant comporter un ou plusieurs hétéroatomes N ou O, mais ne porte pas de fonction acide carboxylique,
▪ R₅ est soit un atome d'hydrogène, soit une chaine comprenant 1 à 6 atomes de carbone ladite chaîne pouvant comporter un ou plusieurs hétéroatomes N ou O, mais ne porte pas de fonction acide carboxylique,
▪ X⁻ est un anion choisi dans le groupe constitué des halogénures, des carboxylates, des sulfates, phosphates et sulfonates,
▪ les atomes de carbone asymétriques présentent une configuration R ou S.

5. Utilisation selon l'une quelconque des revendications 1 à 3, **caractérisée en ce que** le composé est choisi parmi les composés de formule V : 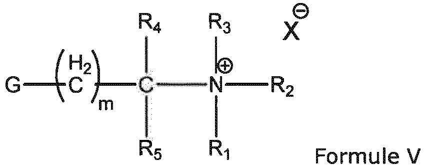 dans laquelle :
▪ G est un groupement phényle ou alkylphényle en C6 à C10
▪ m est un entier égal à 0 ou 1,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ R₄, est une chaîne comprenant 1 à 6 atomes de carbone, ladite chaine pouvant comporter un ou plusieurs hétéroatomes N ou O, mais ne porte pas de fonction acide carboxylique,
▪ R₅ est soit un atome d'hydrogène, soit une chaîne comprenant 1 à 6 atomes de carbone, ladite chaine pouvant comporter un ou plusieurs hétéroatomes N ou O, mais ne porte pas de fonction acide carboxylique,
▪ X⁻ est un anion choisi dans le groupe constitué des halogènures, acétates, sulfates, phosphates et sulfonates,
▪ les atomes de carbone asymétriques présentent une configuration R ou S.

6. Utilisation selon l'une quelconque des revendications 1 à 3, **caractérisée en ce que** le composé est choisi parmi les composés de formule XII : 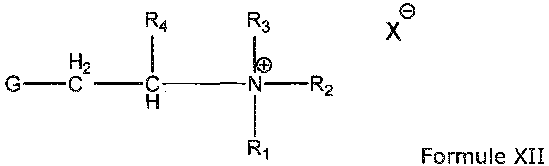 dans laquelle :
▪ G est un groupe phényle ou alkylphényle en C6 à C10, le groupe phényle pouvant être substitué par un ou plusieurs groupements hydroxyles,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ R₄, est une chaîne comprenant 1 à 6 atomes de carbone, ladite chaine pouvant comporter un ou plusieurs hétéroatomes N ou O, mais ne porte pas de fonction acide carboxylique,
▪ X⁻ est au moins un anion choisi dans le groupe constitué des halogènures, des carboxylates, des sulfates, phosphates et sulfonates,
▪ Les atomes de carbone asymétriques présentent une configuration R ou S.

7. Utilisation selon l'une quelconque des revendications 1 à 3, **caractérisée en ce que** le composé est choisi parmi les composés de formule XIII : 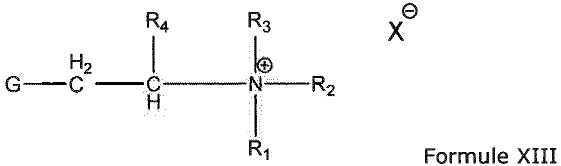 dans laquelle :
▪ G est un groupe phényle ou alkylphényle en C6 à C10, le groupe phényle pouvant être substitué par un ou plusieurs groupements hydroxyles,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ R₄, est une chaîne comprenant 1 à 6 atomes de carbone, ladite chaine comportant un ou plusieurs hétéroatomes N ou O, une fonction amine-N-oxyde, mais ne porte pas de fonction acide carboxylique,
▪ X⁻ est au moins un anion choisi dans le groupe constitué des halogènures, des carboxylates, des sulfates, phosphates et sulfonates,
▪ Les atomes de carbone asymétriques présentent une configuration R ou S.

8. Utilisation selon l'une quelconque des revendications 1 à 3, **caractérisée en ce que** le composé est choisi parmi les composés de formule XIV : 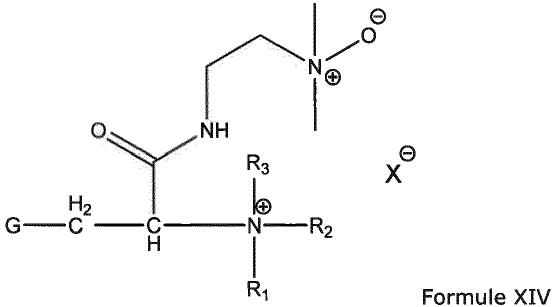 dans laquelle :
▪ G est un groupe phényle ou alkylphényle en C6 à C10, le groupe phényle pouvant être substitué par un ou plusieurs groupements hydroxyles,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ X⁻ est au moins un anion choisi dans le groupe constitué des halogènures, des carboxylates, des sulfates, phosphates et sulfonates,
▪ Les atomes de carbone asymétriques présentent une configuration R ou S.

9. Utilisation selon l'une quelconque des revendications 1 à 3, **caractérisée en ce que** le composé est choisi parmi les composés de formule XV : 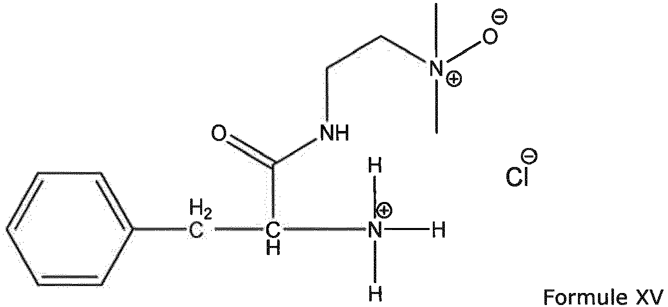 dans laquelle :
▪ Les atomes de carbone asymétriques présentent une configuration R ou S.

10. Utilisation selon l'une quelconque des revendications 1 à 3, **caractérisée en ce que** le composé est choisi parmi les composés de formule VII : 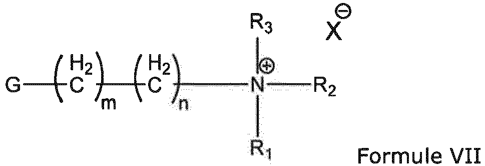 dans laquelle :
▪ G est un groupement phényle ou alkylphényle en C6 à C10, le le groupe phényle pouvant être substitué par un ou plusieurs groupements hydroxyles,
▪ m et n sont des entiers identiques ou différents compris entre 0 et 2 tel que 0 < m + n ≤ 3,
▪ R₁, R₂, R₃, identiques ou différents, sont soit un atome d'hydrogène, soit une chaine comprenant 1 à 4 atomes de carbone, ladite chaine pouvant comporter un ou plusieurs hétéroatomes N ou O,
▪ X⁻ est un anion choisi dans le groupe constitué des halogénures, des carboxylates, des sulfates, phosphates et sulfonates,
▪ les atomes de carbone asymétriques présentent une configuration R ou S.

11. Procédé d'abaissement de la viscosité, **caractérisé en ce que** l'on prépare une solution comprenant un composé de formule I et une protéine comportant au moins un fragment d'anticorps dont la concentration est comprise en 50 et 350 mg/mL et dont le pH est compris entre 5 et 8.

12. Procédé selon la revendication précédente, **caractérisé en ce qu'**est ajouté à une solution difficilement injectable, un composé de formule I et une protéine comportant au moins un fragment d'anticorps dont la concentration est comprise en 50 et 350 mg/mL et dont le pH est compris entre 5 et 8, qui abaisse la viscosité de cette solution d'une valeur d'au moins 15% par rapport à la viscosité d'une solution d'au moins une protéine comportant au moins un fragment d'anticorps de même concentration et de même pH ne contenant pas ledit composé.

13. Procédé selon la revendication 11 ou 12, **caractérisé en ce que** la concentration du composé de formule I dans la formulation finale est comprise entre 100 et 200 mM.

14. Composé réducteur de viscosité de formule XIV : 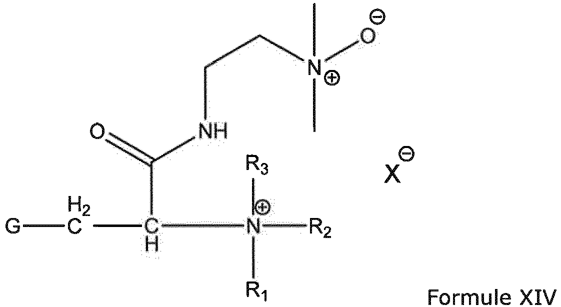 dans laquelle :
▪ G est un groupe phényle ou alkylphényle en C6 à C10, le groupe phényle pouvant être substitué par un ou plusieurs groupements hydroxyles,
▪ R₁, R₂, R₃, identiques ou différents, sont soit un atome d'hydrogène, soit une chaine comprenant 1 à 4 atomes de carbone, ladite chaîne pouvant comporter un ou plusieurs hétéroatomes N ou O,
▪ X⁻ est au moins un anion choisi dans le groupe constitué des halogènures, des carboxylates, des sulfates, phosphates et sulfonates,
▪ Les atomes de carbone asymétriques présentent une configuration R ou S.

15. Composé réducteur de viscosité de formule XV : 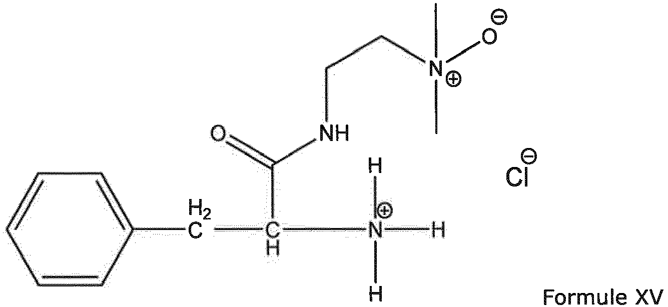 dans laquelle :
▪ Les atomes de carbone asymétriques présentent une configuration R ou S.

16. Composition aqueuse (à viscosité reduite) **caractérisée en ce qu'**elle contient un composé de formule XIV tel que défini dans la revendication 14, et une protéine comportant au moins un fragment d'anticorps dont la concentration est comprise en 50 et 350 mg/mL et dont le pH est compris entre 5 et 8.

17. Composition aqueuse (à viscosité reduite) **caractérisée en ce qu'**elle contient un composé de formule XV tel que défini dans la revendication 15, et une protéine comportant au moins un fragment d'anticorps dont la concentration est comprise en 50 et 350 mg/mL et dont le pH est compris entre 5 et 8.

## Patentansprüche

1. Verwendung einer Verbindung der Formel I mit einer Konzentration in der finalen Formulierung zwischen 10 und 250 mM: 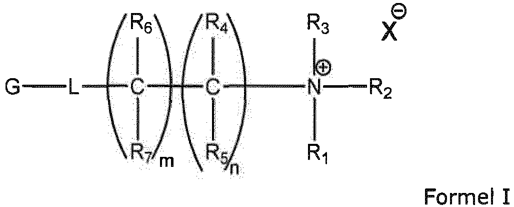 wobei:
• G eine Phenyl- oder Alkylphenylgruppe aus C6 bis C10 ist, wobei die Phenylgruppe mit einer oder mehreren Hydroxylgruppen substituiert sein kann,
• L eine Bindung oder eine Funktion ist, die ausgewählt ist aus der Gruppe bestehend aus der Etherfunktion, der Carbamatfunktion und der Amidfunktion,
• m und n identische oder verschiedene ganze Zahlen zwischen 0 und 2 sind, so dass 0 < m + n ≤ 3 ist,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• R₄, R₅, R₆, und R₇, die identisch oder verschieden sein können, entweder ein Wasserstoffatom oder eine Kette umfassend 1 bis 6 Kohlenstoffatome sind, aber keine Carbonsäurefunktion tragen,
• X⁻ ein Anion ist, das aus der Gruppe ausgewählt ist bestehend aus Haliden, Carboxylaten, Sulfaten, Phosphaten und Sulfonaten,
• die asymmetrischen Kohlenstoffatome eine R- oder S-Konfiguration aufweisen.
wobei die Verbindung eine Löslichkeit in Wasser von ≥ 20 mM bei einem pH-Wert zwischen 5 und 8 aufweist,
zum Verringern der Viskosität einer schwer injizierbaren Lösung von mindestens einem Protein umfasssend wenigstens ein Antikörperfragment, dessen Konzentration zwischen 50 und 350 mg/ml und dessen pH-Wert zwischen 5 und 8 beträgt, um einen Wert von mindestens 15% bezogen auf die Viskosität einer Lösung von mindestens einem Protein umfassend wenigstens ein Antikörperfragment der gleichen Konzentration und des gleichen pH-Wertes, die nicht die Verbindung umfasst.

2. Verwendung nach Anspruch 1, **dadurch gekennzeichnet, dass** das wenigstens ein Antikörperfragment umfassende Protein ein monoklonaler Antikörper ist.

3. Verwendung nach Anspruch 1, **dadurch gekennzeichnet, dass** die Verbindung ausgewählt ist aus den Verbindungen der Formel I, in der wenigstens eine der Gruppen R₄, R₅, R₆ oder R₇ kein Wasserstoffatom ist und eine Amin-N-Oxid-Funktion umfasst.

4. Verwendung nach einem der Ansprüche 1 bis 3, **dadurch gekennzeichnet, dass** die Verbindung ausgewählt ist aus den Verbindungen der Formel II: 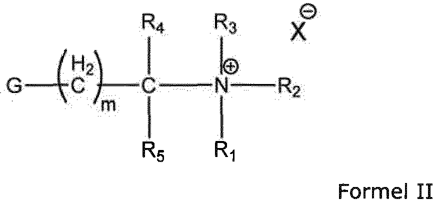 wobei:
• G eine Phenyl- oder Alkylphenylgruppe aus C6 bis C10 ist, wobei die Phenylgruppe mit einer oder mehreren Hydroxylgruppen substituiert sein kann,
• m eine ganze Zahl von 0 oder 1 ist,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• R₄ eine Kette ist umfassend 1 bis 6 Kohlenstoffatome, wobei die Kette ein oder mehrere Heteroatome N oder O umfassen kann, aber keine Carbonsäurefunktion trägt,
• R₅ ein Wasserstoffatom oder eine Kette umfassend 1 bis 6 Kohlenstoffatome ist, wobei die Kette ein oder mehrere Heteroatome N oder O umfassen kann, aber keine Carbonsäurefunktion trägt,
• X⁻ ein Anion ist, das ausgewählt ist aus der Gruppe bestehend aus Haliden, Carboxylaten, Sulfaten, Phosphaten und Sulfonaten,
• die asymmetrischen Kohlenstoffatome eine R- oder S-Konfiguration aufweisen.

5. Verwendung nach einem der Ansprüche 1 bis 3, **dadurch gekennzeichnet, dass** die Verbindung ausgewählt ist aus den Verbindungen der Formel V: 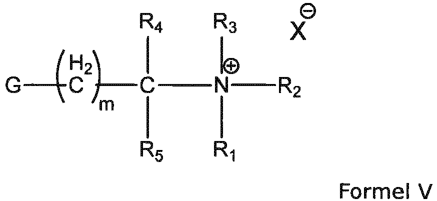 wobei:
• G eine Phenylgruppe oder Alkylphenylgrupp aus C6 bis C10 ist,
• m eine ganze Zahl von 0 oder 1 ist,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• R₄ eine Kette umfassend 1 bis 6 Kohlenstoffatome ist, wobei die Kette ein oder mehrere Heteroatome N oder O umfassen kann, aber keine Carbonsäurefunktion trägt,
• R₅ ein Wasserstoffatom oder eine Kette umfassend 1 bis 6 Kohlenstoffatome ist, wobei die Kette ein oder mehrere Heteroatome N oder O tragen kann, aber keine Carbonsäurefunktion trägt,
• X⁻ ein Anion ist, das ausgewählt ist aus der Gruppe bestehend aus Haliden, Acetaten, Sulfaten, Phosphaten und Sulfonaten,
• die asymmetrischen Kohlenstoffatome eine R- oder S-Konfiguration aufweisen.

6. Verwendung nach einem der Ansprüche 1 bis 3, **dadurch gekennzeichnet, dass** die Verbindung ausgewählt ist aus den Verbindungen der Formel XII: 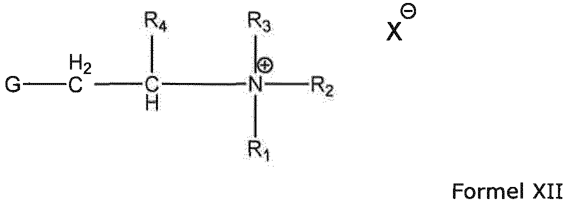 wobei:
• G eine Phenyl- oder Alkylphenylgruppe aus C6 bis C10 ist, wobei die Phenylgruppe mit einer oder mehreren Hydroxylgruppen substituiert sein kann,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• R₄ eine Kette umfassend 1 bis 6 Kohlenstoffatome ist, wobei die Kette ein oder mehrere Heteroatome N oder O umfassen kann, aber keine Carbonsäurefunktion trägt,
• X⁻ wenigstens ein Anion ist, das ausgewählt ist aus der Gruppe bestehend aus Haliden Carboxylaten, Sulfaten, Phosphaten und Sulfonaten,
• die asymmetrischen Kohlenstoffatome eine R- oder S-Konfiguration aufweisen.

7. Verwendung nach einem der Ansprüche 1 bis 3, **dadurch gekennzeichnet, dass** die Verbindung ausgewählt ist aus den Verbindungen der Formel XIII: 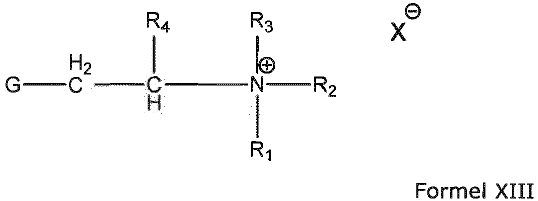 wobei:
• G eine Phenyl- oder Alkylphenylgruppe aus C6 bis C10 ist, wobei die Phenylgruppe mit einer oder mehreren Hydroxylgruppen substituiert sein kann,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• R₄ eine Kette umfassend 1 bis 6 Kohlenstoffatome ist, wobei die Kette ein oder mehrere Heteroatome N oder O, eine Amin-N-Oxid-Funktion umfassen kann, aber keine Carbonsäurefunktion trägt,
• X⁻ wenigstens ein Anion ist, das ausgewählt ist aus der Gruppe bestehend aus Haliden, Carboxylaten, Sulfaten, Phosphaten und Sulfonaten,
• die asymmetrischen Kohlenstoffatome eine R- oder S-Konfiguration aufweisen.

8. Verwendung nach einem der Ansprüche 1 bis 3, **dadurch gekennzeichnet, dass** die Verbindung ausgewählt ist aus den Verbindungen der Formel XIV: 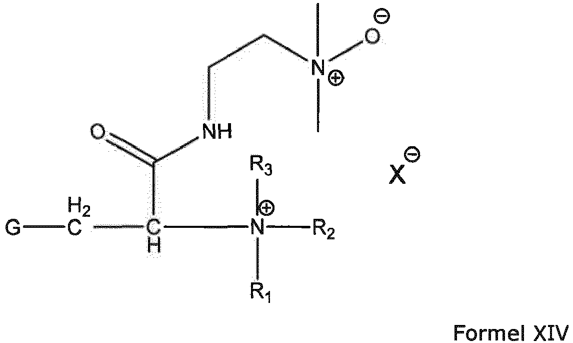 wobei:
• G eine Phenyl- oder Alkylphenylgruppe aus C6 bis C10 ist, wobei die Phenylgruppe mit einer oder mehreren Hydroxylgruppen substituiert sein kann,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• X⁻ wenigstens ein Anion ist, das ausgewählt ist aus der Gruppe bestehend aus Haliden, Carboxylaten, Sulfaten, Phosphaten und Sulfonaten,
• die asymmetrischen Kohlenstoffatome eine R- oder S-Konfiguration aufweisen.

9. Verwendung nach einem der Ansprüche 1 bis 3, **dadurch gekennzeichnet, dass** die Verbindung ausgewählt ist aus den Verbindungen der Formel XV: 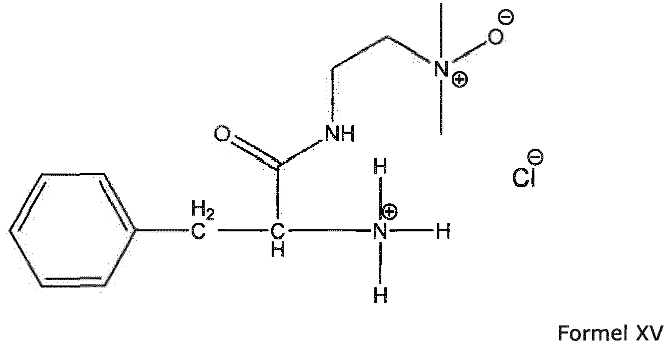 wobei:
• die asymmetrischen Kohlenstoffatome eine R- oder S-Konfiguration aufweisen.

10. Verwendung nach einem der Ansprüche 1 bis 3, **dadurch gekennzeichnet, dass** die Verbindung ausgewählt ist aus den Verbindungen der Formel VII: 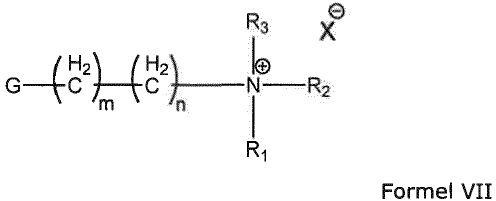 wobei:
• G eine Phenyl- oder Alkylphenylgruppe aus C6 bis C10 ist, wobei die Phenylgruppe mit einer oder mehreren Hydroxylgruppen substituiert sein kann,
• m und n identische oder verschiedene ganze Zahlen zwischen 0 und 2 sind, so dass 0 < m + n ≤ 3,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• X⁻ ein Anion ist, das ausgewählt ist aus der Gruppe bestehend aus Haliden, Carboxylaten, Sulfaten, Phosphaten und Sulfonaten,
• die asymmetrischen Kohlenstoffatome eine R- oder S-Konfiguration aufweisen.

11. Verfahren zur Verringerung von Viskosität, **dadurch gekennzeichnet, dass** eine Lösung hergestellt wird, die eine Verbindung der Formel I und ein Protein umfassend wenigstens ein Antikörperfragment umfasst, dessen Konzentration zwischen 50 und 350 mg/ml und dessen pH-Wert zwischen 5 und 8 beträgt.

12. Verfahren nach dem vorhergehenden Anspruch, **dadurch gekennzeichnet, dass** zu einer schwer injizierbaren Lösung eine Verbindung der Formel I und ein wenigstens ein Antikörperfragment umfassendes Protein hinzugegeben wird, dessen Konzentration zwischen 50 und 350 mg/ml und dessen pH-Wert zwischen 5 und 8 beträgt, die die Viskosität dieser Lösung um einen Wert von wenigstens 15 % verringert bezogen auf die Viskosität einer Lösung wenigstens eines, wenigstens ein Antikörperfragment umfassenden Proteins der gleichen Konzentration und des gleich pH-Wertes, die nicht die Verbindung enthält.

13. Verfahren nach Anspruch 11 oder 12, **dadurch gekennzeichnet, dass** die Konzentration der Verbindung nach Formel I in der finalen Formulierung zwischen 100 und 200 mM beträgt.

14. Viskositätsverringernde Verbindung der Formel XIV: 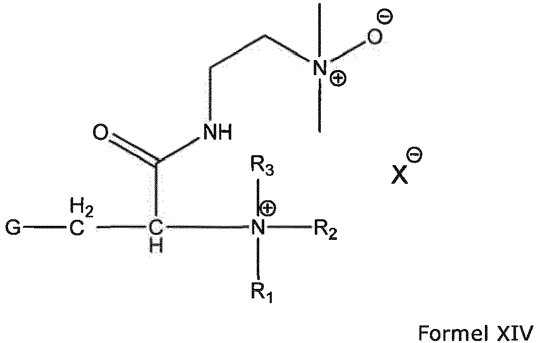 wobei:
• G eine Phenyl- oder Alkylphenylgruppe aus C6 bis C10 ist, wobei die Phenylgruppe mit einer oder mehreren Hydroxylgruppen substituiert sein kann,
• R₁, R₂, und R₃, die identisch oder verschieden sein können, entweder ein Wasserstoffatom oder eine Kette umfassend 1 bis 4 Kohlenstoffatome sind, wobei die Kette ein oder mehrere Heteroatome N oder O umfassen kann,
• X⁻ wenigstens ein Anion ist, das ausgewählt ist aus der Gruppe bestehend aus Haliden, Carboxylaten, Sulfaten, Phosphaten und Sulfonaten,
• die asymmetrischen Kohlenstoffatome eine R- oder S-Konfiguration aufweisen.

15. Viskositätsverringernde Verbindung der Formel XV: 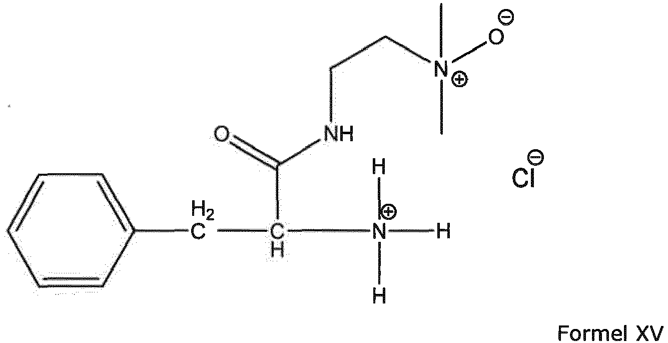 wobei:
• die asymmetrischen Kohlenstoffatome eine R- oder S-Konfiguration aufweisen.

16. Wässrige Zusammensetzung (mit veringerter Viskosität), **dadurch gekennzeichnet, dass** sie eine Verbindung der Formel XIV, wie in Anspruch 14 definiert, und ein wenigstens ein Antikörperfragment umfassendes Protein enthält, dessen Konzentration zwischen 50 und 350 mg/ml und dessen pH-Wert zwischen 5 und 8 beträgt.

17. Wässrige Zusammensetzung (mit verringerter Viskosität), **dadurch gekennzeichnet, dass** sie eine Verbindung der Formel XV, wie in Anspruch 15 definiert, und ein wenigstens ein Antikörperfragment umfassendes Protein enthält, dessen Konzentration 50 bis 350 mg/ml und dessen pH-Wert 5 bis 8 beträgt.

## Claims

1. Use of a compound of formula I, at a concentration in the final formulation comprised between 10 and 250 mM: 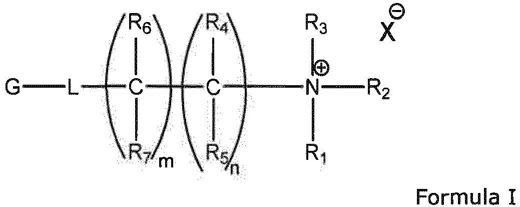 wherein:
• G is a C6-C10 phenyl or alkylphenyl group, the phenyl group being optionally substituted by one or more hydroxyl groups,
• L is either a bond or a function selected from the group consisting of the ether function, the carbonate function or the amide function,
• m and n are identical or different whole numbers between 0 and 2, such that 0 < m + n ≤ 3,
• R₁, R₂, R₃, identical or different, are either a hydrogen atom or a chain including 1 to 4 carbon atoms, said chain optionally comprising one or more heteroatoms N or O,
• R₄, R₅, R₆, and R₇, identical or different, are either a hydrogen atom or a chain including 1 to 6 carbon atoms, but not bearing a carboxylic acid function,
• X⁻ is an anion selected from the group consisting of the halides, the carboxylates, the sulfates, phosphates and sulfonates,
• the asymmetric carbon atoms have an R or S configuration.
said compound having a solubility in water ≥ 20 mM at a pH between 5 and 8,
in order to lower the viscosity of a difficult-to-inject solution of at least one protein comprising at least one antibody fragment the concentration of which is between 50 and 350 mg/mL and the pH of which is between 5 and 8, by a value of at least 15% with respect to the viscosity of a solution of at least one protein comprising at least one antibody fragment, having the same concentration and the same pH and not containing said compound.

2. Use according to Claim 1, **characterized in that** the protein comprising at least one antibody fragment is a monoclonal antibody.

3. Use according to Claim 1, **characterized in that** the compound is selected from the compounds of formula I in which at least one group R₄, R₅, R₆ or R₇ is not a hydrogen atom and includes an amine-N-oxide function.

4. Use according to any one of Claims 1 to 3, **characterized in that** the compound is selected from the compounds of formula II: 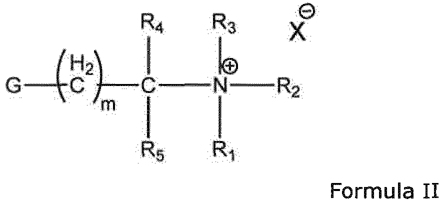 wherein:
• G is a C6-C10 phenyl or alkylphenyl group, the phenyl group being optionally substituted by one or more hydroxyl groups,
• m is a whole number equal to 0 or 1,
• R₁, R₂, R₃, identical or different, are either a hydrogen atom or a chain including 1 to 4 carbon atoms, said chain optionally comprising one or more heteroatoms N or O,
• R₄ is a chain including from 1 to 6 carbon atoms, said chain optionally comprising one or more heteroatoms N or O, but not bearing a carboxylic acid function,
• R₅ is either a hydrogen atom or a chain including 1 to 6 carbon atoms, said chain optionally comprising one or more heteroatoms N or O, but not bearing a carboxylic acid function,
• X⁻ is an anion selected from the group consisting of the halides, the carboxylates, the sulfates, phosphates and sulfonates,
• the asymmetric carbon atoms have an R or S configuration.

5. Use according to any one of Claims 1 to 3, **characterized in that** the compound is selected from the compounds of formula V: 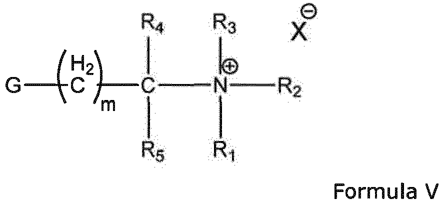 wherein:
• G is a C6-C10 phenyl or alkylphenyl group,
• m is a whole number equal to 0 or 1,
• R₁, R₂, R₃, identical or different, are either a hydrogen atom or a chain including 1 to 4 carbon atoms, said chain optionally comprising one or more heteroatoms N or O,
• R₄ is a chain including 1 to 6 carbon atoms, said chain optionally comprising one or more heteroatoms N or O, but not bearing a carboxylic acid function,
• R₅ is either a hydrogen atom or a chain including 1 to 6 carbon atoms, said chain optionally comprising one or more heteroatoms N or O, but not bearing a carboxylic acid function,
• X⁻ is an anion selected from the group consisting of the halides, acetates, sulfates, phosphates and sulfonates,
• the asymmetric carbon atoms have an R or S configuration.

6. Use according to any one of Claims 1 to 3, **characterized in that** the compound is selected from the compounds of formula XII: 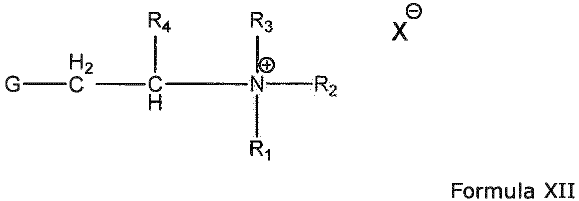 wherein:
• G is a C6-C10 phenyl or alkylphenyl group, the phenyl group being optionally substituted by one or more hydroxyl groups,
• R₁, R₂, R₃, identical or different, are either a hydrogen atom or a chain including 1 to 4 carbon atoms, said chain optionally comprising one or more heteroatoms N or O,
• R₄ is a chain including 1 to 6 carbon atoms, said chain optionally comprising one or more heteroatoms N or O, but not bearing a carboxylic acid function,
• X⁻ is at least one anion selected from the group consisting of the halides, the carboxylates, the sulfates, phosphates and sulfonates,
• the asymmetric carbon atoms have an R or S configuration.

7. Use according to any one of Claims 1 to 3, **characterized in that** the compound is selected from the compounds of formula XIII: 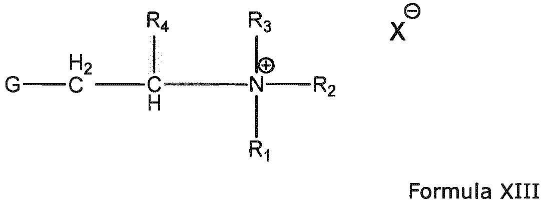 wherein:
• G is a C6-C10 phenyl or alkylphenyl group, the phenyl group being optionally substituted by one or more hydroxyl groups,
• R₁, R₂, R₃, identical or different, are either a hydrogen atom or a chain including 1 to 4 carbon atoms, said chain optionally comprising one or more heteroatoms N or O,
• R₄ is a chain including 1 to 6 carbon atoms, said chain comprising one or more heteroatoms N or O, an amine N-oxide function, but not bearing a carboxylic acid function,
• X⁻ is at least one anion selected from the group consisting of the halides, the carboxylates, the sulfates, phosphates and sulfonates,
• the asymmetric carbon atoms have an R or S configuration.

8. Use according to any one of Claims 1 to 3, **characterized in that** the compound is selected from the compounds of formula XIV: 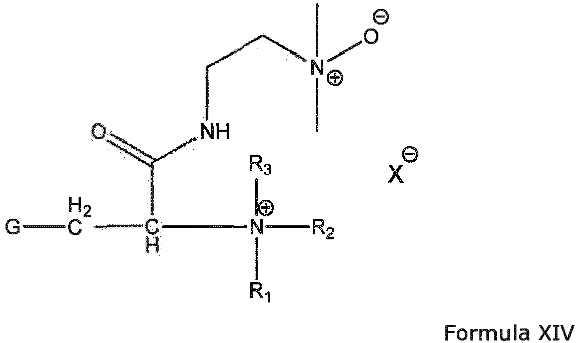 wherein:
• G is a C6-C10 phenyl or alkylphenyl group, the phenyl group being optionally substituted by one or more hydroxyl groups,
• R₁, R₂, R₃, identical or different, are either a hydrogen atom or a chain including 1 to 4 carbon atoms, said chain optionally comprising one or more heteroatoms N or O,
• X⁻ is at least one anion selected from the group consisting of the halides, the carboxylates, the sulfates, phosphates and sulfonates,
• the asymmetric carbon atoms have an R or S configuration.

9. Use according to any one of Claims 1 to 3, **characterized in that** the compound is selected from the compounds of formula XV: 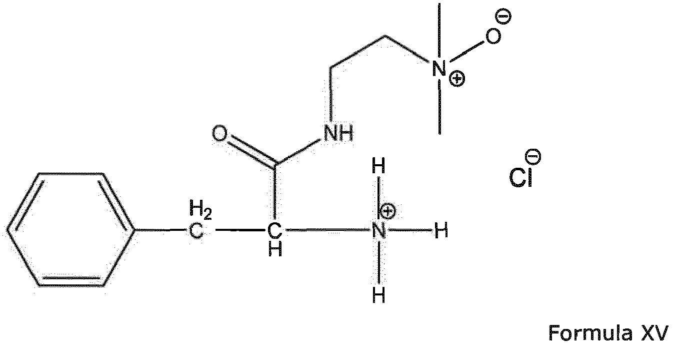 wherein:
• the asymmetric carbon atoms have an R or S configuration.

10. Use according to any one of Claims 1 to 3, **characterized in that** the compound is selected from the compounds of formula VII: 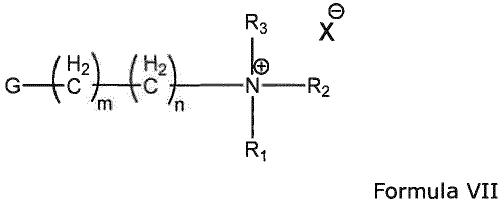 wherein:
• G is a C6-C10 phenyl or alkylphenyl group, the phenyl group being optionally substituted by one or more hydroxyl groups,
• m and n are identical or different whole numbers between 0 and 2, such that 0 < m + n ≤ 3,
• R₁, R₂, R₃, identical or different, are either a hydrogen atom or a chain including 1 to 4 carbon atoms, said chain optionally comprising one or more heteroatoms N or O,
• X⁻ is an anion selected from the group consisting of the halides, the carboxylates, the sulfates, phosphates and sulfonates,
• the asymmetric carbon atoms have an R or S configuration.

11. Method for lowering the viscosity, **characterized in that** a solution is prepared, which includes a compound of formula I and a protein comprising at least one antibody fragment, the concentration of which is between 50 and 350 mg/mL and the pH of which is between 5 and 8.

12. Method according to the preceding claim, **characterized in that** the following are added to a difficult-to-inject solution: a compound of formula I and a protein comprising at least one antibody fragment, the concentration of which is between 50 and 350 mg/mL and the pH of which is between 5 and 8, which lowers the viscosity of this solution by a value of at least 15% with respect to the viscosity of a solution of at least one protein comprising at least one antibody fragment, having the same concentration and the same pH, and not containing said compound.

13. Method according to Claim 11 or 12, **characterized in that** the concentration of the compound of formula I in the formulation is between 100 and 200 mM.

14. Viscosity reducing compound of formula XIV: 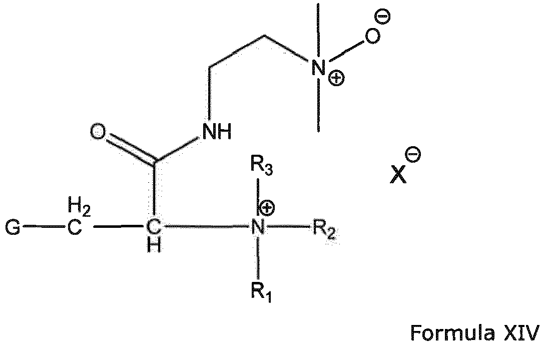 wherein:
• G is a C6-C10 phenyl or alkylphenyl group, the phenyl group being optionally substituted by one or more hydroxyl groups,
• R₁, R₂, R₃, identical or different, are either a hydrogen atom or a chain including 1 to 4 carbon atoms, said chain optionally comprising one or more heteroatoms N or O,
• X⁻ is at least one anion selected from the group consisting of the halides, the carboxylates, the sulfates, phosphates and sulfonates,
• the asymmetric carbon atoms have an R or S configuration.

15. Viscosity reducing compound of formula XV: 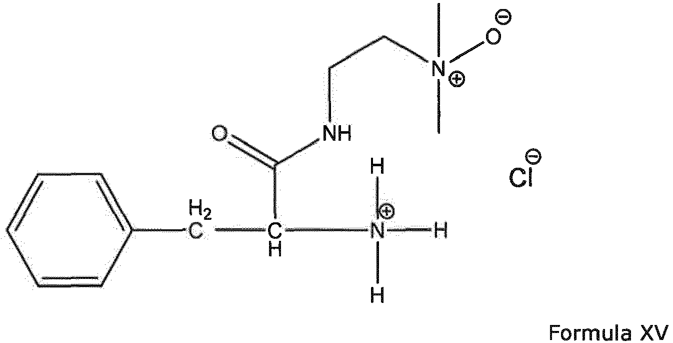 wherein:
• the asymmetric carbon atoms have an R or S configuration.

16. Aqueous composition (having a reduced viscosity), **characterized in that** it contains a compound of formula XIV as defined in Claim 14 and a protein comprising at least one antibody fragment, the concentration of which is between 50 and 350 mg/mL and the pH of which is between 5 and 8.

17. Aqueous composition (having a reduced viscosity), **characterized in that** it contains a compound of formula XV as defined in Claim 15 and a protein comprising at least one antibody fragment, the concentration of which is between 50 and 350 mg/mL and the pH of which is between 5 and 8.
